(19) Europäisches Patentamt
European Patent Office
Office européen des brevets

(11) Numéro de publication: **0 172 083 B1**

(12) # FASCICULE DE BREVET EUROPEEN

(45) Date de publication du fascicule du brevet: **20.04.88**

(21) Numéro de dépôt: **85401480.0**

(22) Date de dépôt: **18.07.85**

(51) Int. Cl.⁴: **C 07 D 471/04**, A 61 K 31/435, C 07 D 215/38, A 61 K 31/47, C 07 D 401/12, C 07 D 409/12, C 07 D 405/12 // (C07D471/04, 221:00, 221:00)

(54) **Nouveaux amides substitués, leur préparation et les médicaments qui les contiennent.**

(30) Priorité: **19.07.84 FR 8411442**

(43) Date de publication de la demande: **19.02.86 Bulletin 86/8**

(45) Mention de la délivrance du brevet: **20.04.88 Bulletin 88/16**

(84) Etats contractants désignés: **AT BE CH DE FR GB IT LI LU NL SE**

(56) Documents cités:
**WO - A - 84/00489**
**DE - A - 1 923 047**
**FR - A - 1 006 725**
**US - A - 3 389 142**
**US - A - 3 624 097**

(73) Titulaire: **RHONE-POULENC SANTE, Les Miroirs 18 Avenue d'Alsace, F-92400 Courbevoie Cédex (FR)**

(72) Inventeur: **Cotrel, Claude, 17 A, avenue du Docteur Arnold Netter, F-75012 Paris (FR)**
Inventeur: **Guyon, Claude, 17 bis avenue Henri Martin, F-94100 Saint-Maur des Fosses (FR)**
Inventeur: **Roussel, Gérard, 20 ter rue des Carrières, F-91450 Soisy sur Seine (FR)**
Inventeur: **Taurand, Gérard, 13 Passage Saillenfait, F-94000 Creteil (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 172 083 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

Dans la littérature ont été décrits:

— des procédés pour la préparation de N-(quinolyl-2) benzamide et son oxyde: M.A. Solekhova et Yu.V. Kurbatov Fiz. Khim. Issled. Sintetich. i Prirod Soedin., Samarkand, 10-16 (1980) [C.A. 96 142 655 y], M.A. Solekhova et Yu. V. Kurbatov, Zh. Org. Khim., 17 (5), 1121 (1981) [C.A. 95 187 028s] et Tamara et coll. Chem. Pharm. Bull., 19 (6), 1285-6 (1971),

— des dérivés de benzamides utiles comme antiarythmiques de formule générale:

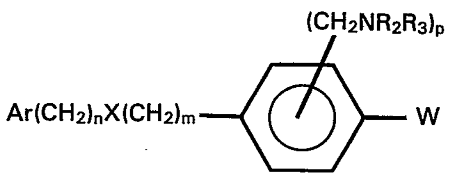

demande de brevet PCT 8 400 489,

— des dérivés de naphthyridines-1,8 utiles comme bronchodilatateurs et vasodilatateurs périphériques ou comme agents hypotenseurs, de structure:

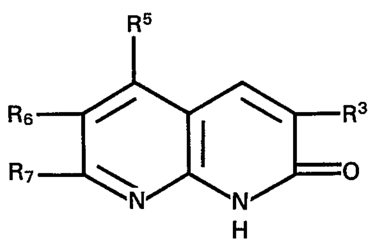

demande de brevet néerlandais 7 305 482 et brevet américain 3 993 656.

La présente invention concerne de nouveaux amides substitués répondant à la formule générale:

R-CONH-Het (I)

leur préparation et les compositions pharmaceutiques qui les contiennent.

Dans la formule générale (I), le symbole R représente un radical cycloalcoyle contenant 3 à 6 atomes de carbone, cyclohexadiényle, phényle, phényle substitué par 1 ou 2 atomes de fluor ou par un radical hydroxy, ou substitué en position -3 ou -4 par un radical alcoyle ou alcoyloxy, ou en positions -3 et -4 par un radical méthylènedioxy, ou substitué en position -2 ou -3 par un radical dialcoylamino, ou bien le symbole R représente un radical hétérocyclyle choisi parmi pyridyle-3, alcoyloxypyridyle-3, thiényle, alcoylthiényle, furyle, tétrahydropyridyle, pyridazinyle et alcoylpyridazinyle, et le symbole Het représente un radical quinolyle-2 ou naphtyridine-1,8 yle-2 éventuellement substitués en position -7 par un atome d'halogène, par un radical hydroxyméthyle, par un radical alcoyle, alcoyloxy, alcoyloxyalcoyloxy, par un radical alcényloxy ou alcynyloxy contenant 3 ou 4 atomes de carbone, par un radical alcoylthio ou benzylthio, par un radical phénoxy (éventuellement substitué par un atome de fluor ou substitué par un atome de chlore ou de brome en position -2, ou substitué par un radical alcoyloxy ou par 1 ou 2 radicaux alcoyle) ou par un radical pyridyloxy ou alcoylpipéridyloxy, étant entendu que, lorsque Het représente un radical quinolyle-2, R est autre que phényle et que les radicaux ou portions alcoyles cités ci-dessus sont droits ou ramifiés et contiennent 1 à 4 atomes de carbone.

Lorsque le symbole Het comporte un substituant halogène, ce dernier est choisi parmi le fluor, le chlore et le brome.

Selon l'invention, les produits de formule générale (I) peuvent être préparés par action d'un acide de formule générale:

R-COOH (II)

dans laquelle R est défini comme précédemment, ou d'un dérivé réactif de cet acide, sur une amine de formule générale:

$H_2N$-Het (III)

dans laquelle Het est défini comme précédemment.

Il est entendu que, lorsque l'acide de formule générale (II) contient un radical hydroxy, ce dernier est préalablement protégé, par toute méthode connue qui n'altère pas le reste de la molécule. A titre d'exemple, il est notamment protégé par un radical acétyle qui peut être éliminé par traitement en milieu basique, par exemple par traitement par la potasse éthanolique.

De même lorsque dans l'amine de formule générale (III) le symbole Het contient un substituant hydroxyalcoyle il est préférable de protéger le radical hydroxy préalablement à la réaction. La protection s'effectue par toute méthode connue en soi qui n'altère pas le reste de la molécule, notamment au moyen d'un radical tétrahydropyrannyle qui peut être éliminé facilement par acidolyse en milieu aqueux.

Lorsque l'on met en œuvre l'acide de formule générale (II), on opère en présence d'un agent de condensation peptidique tel qu'un carbodiimide (par exemple le dicyclohexylcarbodiimide) ou le N,N'-carbonyldiimidazole ou l'éthyoxy-2 éthoxycarbonyl-1 dihydro-1,2 quinoléine, dans un solvant organique tel qu'un éther (par exemple tétrahydrofuranne, dioxanne, glyme, diglyme), un amide (par exemple diméthylformamide), un nitrile (par exemple acétonitrile) ou un solvant chloré (par exemple chlorure de méthylène, dichloréthane ou chloroforme) à une température comprise entre 0°C et la température de reflux du mélange réactionnel. De préférence on opère à 20°C.

Lorsque l'on met en œuvre un dérivé réactif de l'acide de formule générale (II) il est possible de faire réagir l'anhydride, un anhydride mixte, un halogénure d'acide ou un ester [qui peut être choisi parmi les esters activés ou non de l'acide de formule générale (II)]. On opère alors soit en milieu organique, éventuellement en présence d'un accepteur d'acide tel qu'une base organique azotée (par exemple une tri-

alcoylamine, une pyridine, le diaza-1,8 bicyclo [5.4.0] undecène-7 ou le diaza-1,5 bicyclo [4.3.0] nonène-5), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino terreux à une température comprise entre 0 et 40°C. Il est également possible d'opérer sans solvants à la température de fusion du mélange réactionnel.

Selon l'invention, les produits de formule générale (I) dans laquelle le symbole Het contient un substituant hydroxyméthyle, peuvent également être préparés par réduction de l'aldéhyde correspondant.

La réduction s'effectue avantageusement par action du borohydrure de sodium ou par hydrogénation catalytique en présence de nickel, de platine ou de palladium en milieu organique ou hydroorganique, par exemple dans un alcool (méthanol, éthanol) dans un éther (tétrahydrofuranne, dioxanne) ou dans un mélange de ces solvants à une température comprise entre 0 et 50°C. L'aldéhyde de départ peut être préparé par oxydation d'un produit de formule générale (I) dans laquelle Het contient un substituant méthyle. La réaction s'effectue au moyen d'un agent d'oxydation tel que l'oxyde de sélénium, dans un solvant organique tel que le dioxanne à la température de reflux du mélange réactionnel.

Les acides de formule générale (II) peuvent être préparés par application des méthodes ci-dessous (ou par analogie avec ces méthodes):

— H.D. HARTOUGH, the Chemistry of heterocyclic compounds, thiophen and its derivatives, Interscience Publishers Inc. New-York, page 363 (1952);

— J.W. MASON, The Chemistry of heterocyclic compounds, Pyridazine carboxylic acids, John Wiley and Sons Inc. New-York, page 407 (1973);

— E.P. OLIVETO, The Chemistry of heterocyclic compounds Pyridine-carboxylic acids, Interscience Publishers Inc., page 179 (1962).

— A.A. PETROV. et coll., Zhur Obshchei Khim, 26, 1958 (1956), M.E. KUEHNE et coll., Org. Synth., 43, 22 (1968), S. Hunig et coll., Chem. Ber., 90, 238 (1957) ou H. Plieninger et coll., Chem. Ber., 94, 2088 (1961) lorsqu'il s'agit d'un acide cyclohexadiénylcarboxylique.

Les amines de formule générale (III) dans laquelle le symbole Het porte un substituant alcoyloxy, alcoyloxyalcoyloxy, alcényloxy, alcynyloxy ou phénoxy éventuellement substitué, un substituant pyridyloxy ou alcoylpipéridyloxy, ou un substituant alcoylthio ou benzylthio, peuvent être préparées à partir de l'amine correspondante dans laquelle le symbole Het porte un substituant halogène (de préférence un atome de chlore) par action respectivement d'un dérivé hydroxylé ou d'un thiol de formules générales:

R'OH (IVa) ou R''SH (IVb)

[dans lesquelles R' représente un radical alcoyle, alcoyloxyalcoyle, alcényle, alcynyle, phényle éventuellement substitué (par un atome de fluor, par un atome de chlore ou de brome en position -2, par un radical alcoyloxy, ou par un ou 2 radicaux alcoyle), ou un radical pyridyle ou alcoylpipéridyle et R'' représente un radical alcoyle ou benzyle], en milieu basique, ou par action de l'alcoolate ou du thiolate correspondant.

La réaction s'effectue généralement en présence d'une base forte (soude, potasse, hydroxyde d'ammonium quaternaire, éthylate de sodium par exemple), à une température comprise entre 50 et 150°C, ou bien en présence de l'alcoolate ou de thiolate correspondant (par exemple l'alcoolate de sodium), dans un solvant tel qu'un amide (diméthylformamide par exemple) ou un éther (tétrahydrofuranne, diméthoxyéthane par exemple), ou sans solvant, en présence d'un excès de dérivé hydroxylé ou de thiol, à une température comprise entre 70°C et la température de reflux du mélange réactionnel.

Lorsque l'on met en œuvre l'alcoolate ou le thiolate, celui-ci est obtenu préalablement par action du sodium sur l'alcool de formule générale (IVa) ou le thiol de formule générale (IVb) à une température comprise entre 20 et 80°C, ou par action de l'hydrure de sodium à une température comprise entre 0 et 20°C dans un solvant tel que le diméthylformamide, le diméthoxyéthane, ou le tétrahydrofuranne. Il n'est pas nécessaire d'isoler l'alcoolate ou le thiolate obtenu pour le mettre en œuvre dans la réaction suivante.

Les amines de formule générale (III) dans laquelle le symbole Het contient un substituant hydroxyméthyle peuvent être obtenues par réduction de l'aldéhyde correspondant dont la fonction amine a été préalablement protégé (par exemple par un radical benzyle). La réaction s'effectue dans les conditions décrites précédemment pour la préparation d'un produit de formule générale (I) dans laquelle le symbole Het porte un substituant hydroxyméthyle. L'aldéhyle de départ peut être préparé par analogie avec la préparation de la matière première dans le procédé ci-dessus.

Les amines de formule générale (III) peuvent également être préparées par application des méthodes décrites ci-après dans les exemples ou par application ou par analogie avec les méthodes décrites par:

— S. CARBONI, Gazz. Chim. Italiana, 96, 1456 (1966);

— E.V. BROWN, J. Org. Chem., 30, 1607 (1965);

— G. JONES, The Chemistry of Heterocyclic compounds, Quinolins, John Wiley and Sons Inc., New-York, Part I (1977), Part II (1982)

— ou selon les brevets américains 3 948 917 et 3 884 921.

Selon l'invention, les produits de formule générale (I) dans laquelle le symbole R est un radical tétrahydroxypyridyle peuvent être préparés par action de la tétrahydropyridine sur un carbamate de formule générale:

$$C_6H_5\text{-O-CO-NH-Het} \qquad (V)$$

dans laquelle Het est défini comme précédemment.

La réaction s'effectue généralement dans un solvant organique tel qu'un nitrile (acétonitrile par exemple) ou un éther (tétrahydrofuranne par exemple) ou dans un mélange de ces solvants, à une température comprise entre 60°C et la température de reflux du mélange réactionnel.

Le carbamate de formule générale (V) peut être obtenu par action d'une amine de formule générale (III) sur le chloroformiate correspondant.

On opère généralement dans la pyridine à une température comprise entre 0 et 40°C en présence d'un accepteur d'acide tel que la pyridine ou une trialcoylamine (triéthylamine par exemple) éventuellement dans un solvant organique tel que le tétrahydrofuranne.

Les nouveaux amides selon la présente invention peuvent être purifiés le cas échéant, par des méthodes physiques telles que la cristallisation ou la chromatographie.

Les produits de formule générale (I) présentent des propriétés pharmacologiques particulièrement intéressantes. Ils manifestent une activité comme anxiolytiques, hypnotiques, anticonvulsivants, antiépileptiques et myorelaxants de bon niveau, mise en évidence dans les tests ci-dessous.

Notamment ils présentent une bonne affinité in vitro pour les sites récepteurs à benzodiazépine à des concentrations comprises entre 5 et des valeurs voisines de 1000 nM selon la technique décrite par J.C. Blanchard et L. Julou, J. of Neurochemistry, 40, 601 (1983) inspirée de travaux de Squires et Braestrup, Nature, 266, 732 (1977);

Chez l'animal (souris) ils se sont montrés actifs à des doses comprises entre 10 et environ 200 mg/kg par voie orale vis-à-vis des convulsions induites par le pentétrazol selon une technique voisine de celle de Everett et Richards, J. Pharmacol., 81, 402 (1944).

De plus les produits de formule générale (I) dans laquelle R est un radical phényle substitué en position −3 ou −4 par un radical alcoyle ou en position −3 et −4 par un radical méthylènedioxy et Het est un radical naphthyridine-1,8 yle-2 substitué en position −7 par un radical tel que défini dans la formule générale (I), ou bien R est un radical phényle et Het est un radical naphthyridine-1,8 yle-2 substitué en position −7 par un radical hydroxyméthyle, alcoyle, benzylthio ou par un radical phénoxy substitué, tel que défini dans la formule générale (I), ou un radical pyridyloxy ou alcoylpipéridyloxy, ou bien R est un radical méthoxyphényle et Het est un radical naphthyridine-1,8 yle-2 substitué en position −7 par un atome de brome ou par un radical hydroxyméthyle, benzylthio ou phénoxy, présentent une activité immunostimulante.

L'activité immunostimulante a été mise en évidence in vitro à des concentrations molaires comprises entre $10^{-5}$ et $10^{-6}$ dans la stimulation de l'activité cytostatique des macrophages péritonéaux de la souris vis-à-vis des cellules tumorales $P_{815}$ selon une technique inspirée de M.I.C. Gyöngyössi et coll., Cell. Immunol., 45, 1 (1979), et in vivo chez la souris, ils stimulent les réactions de défense contre l'infection a Klebsiella pneumoniae à des doses comprises entre 2 et 20 mg/kg i.p. selon une technique inspirée de M.A. Parant et coll., Infect. Immun., 27, 826 (1980).

Un avantage de l'activité immunostimulante des produits est leur utilisation comme agent anti-infectieux ayant notamment la possibilité de lutter contre des germes devenus résistants aux antibiotiques traditionnels.

Par ailleurs, les produites de formule générale (I) dans laquelle le symbole R représente un radical méthoxy-4 phényle, thiényle-3 ou méthyl-5 thiényle et le symbole Het représente un radical naphthyridine-1,8 yle-2 substitué en position −7 par un atome de chlore, un radical méthoxy ou hydroxyméthyle ou bien dans laquelle le symbole R représente un radical thiényle-2 et le symbole Het représente un radical naphthyridine-1,8 yle-2 substitué en position −7 par un radical méthoxy ou phénoxy manifestent également une activité antivirale.

L'activité antivirale a été mise en évidence in vitro sur virus grippaux A et B à des concentrations comprises entre 1,5 et 30 µg/cm$^3$.

En outre, ces produits exercent in vivo une activité protectrice significative vis-à-vis de l'infection grippale ($A_2$ Am Arbor) de la souris, à des doses comprises entre 30 et 300 mg/kg par voie orale.

Par ailleurs les produits selon l'invention présentent une faible toxicité: leur $DL_{50}$ par voie orale chez la souris est comprise entre 300 et 900 mg/kg et des doses supérieures à 900 mg/kg.

D'un intérêt particulier sont les produits de formule générale (I) pour lesquels le symbole R est un radical cyclopropyle ou cyclobutyle, cxclohexadiène-1,4 yle-1, phényle, phényle substitué par un ou 2 atomes de fluor, ou par un radical hydroxy, ou substitué en position −3 ou −4 par un radical alcoyle contenant 1 ou 2 atomes de carbone, ou un radical alcoyloxy contenant 1 à 4 atomes de carbone, ou en positions −3 et −4 par un radical méthylènedioxy ou substitué par un radical diméthylamino en position −3, ou bien le symbole R est un radical hétérocyclyle choisi parmi pyridyle-3, alcoyloxy-6 pyridyle-3, thiényle, alcoyl-5 thiényle, furyle, pyridazinyle-3 ou alcoyl-6 pyridazinyle-3 dont les portions alcoyle contiennent 1 ou 2 atomes de carbone, et le symbole Het représente un radical quinolyle-2 ou naphthyridine-1,8 yle-2 éventuellement substitués en position −7 par un atome d'halogène, par un radical hydroxyméthyle, par un radical alcoyle ou alcoylthio contenant 1 ou 2 atomes de carbone, par un radical alcoyloxy contenant 1 à 4 atomes de carbone ou alcoyloxyalcoyloxy contenant 2 à 4 atomes de carbone, par un radical allyloxy, propargyloxy ou benzylthio, par un radical phénoxy, phénoxy substitué (par un atome de fluor ou par un atome de chlore ou de brome en position −2, par un radical alcoyloxy ou par 1 ou 2 radicaux alcoyle contenant 1 ou 2 atomes de carbone) ou par un radical pyridyl-3 oxy ou alcoyl-1 pipéridyl-4 oxy, étant entendu que lorsque Het représente un radical quinolyle-2, R est autre que phényle.

Et parmi ces produits, plus spécialement actifs, sont les produits de formule générale (I) dans laquelle le symbole R est un radical cyclopropyle, cyclohexadiène-1,4 yle, phényle, phényle substitué par 1 ou 2 atomes de fluor ou par un radical hydroxy, ou substitué en position −3 ou −4 par un radical alcoyloxy contenant 1 ou 2 atomes de carbone ou en

positions -3 et -4 par un radical méthylènedioxy, ou substitué en position -3 par un radical diméthylamino, ou bien le symbole R est un radical hétérocyclyle choisi parmi pyridyle-3, alcoyloxy-6 pyridyle-3, dont la portion alcoyle contient 1 ou 2 atomes de carbone, thiényle ou furyle et le symbole Het représente un radical naphthyridine-1,8 yle-2 éventuellement substitué en position -7 par un atome d'halogène par un radical alcoyle, alcoyloxy ou alcoylthio contenant 1 ou 2 atomes de carbone, par un radical alcoyloxyalcoyloxy contenant 2 à 4 atomes de carbone, par un radical allyloxy ou phénoxy (éventuellement substitué par un atome de fluor, ou par un atome de chlore ou de brome en position -2, par un radical alcoyloxy ou par 1 ou 2 radicaux alcoyle contenant 1 ou 2 atomes de carbone), ou par un radical pyridyl-3 oxy.

Notamment, plus spécialement remarquables sont les produits de formule générale (I) dans laquelle le symbole R est un radical cyclopropyle, phényle, phényle substitué en position -3 ou -4 par un atome de fluor, ou substitué par 2 atomes de fluor ou par un radical hydroxy, ou substitué en position -3 ou -4 par un radical méthoxy, ou bien le symbole R est un radical hétérocyclyle choisi parmi alcoyloxy-6 pyridyle dont la portion acloyle contient 1 ou 2 atomes de carbone, thiényle-2 ou furyle-3, et le symbole Het représente un radical naphthyridine-1,8 yle-2 substitué en position -7 par un atome d'halogène, par un radical méthoxy ou par un radical phénoxy (éventuellement substitué par un atome de fluor ou en position -2 par un atome de chlore ou un radical méthyle, ou en position -2 ou -3 par un radical méthoxy), ou le symbole R représente un radical cyclohexadiène-1,4 yle-1, phényle (substitué en positions -3 et -4 par un radical méthylènedioxy ou substitué en position -3 par un radical diméthylamino) ou un radical pyridyle-3 et le symbole Het représente un radical naphtyridine-1,8 yle-2 substitué en position -7 par un atome d'halogène et parmi ceux-ci les produits suivants:

— N-(méthoxy-7 naphtyridine-1,8 yl-2) cyclopropanecarboxamide
— N-(chloro-7 naphtyridine-1,8 yl-2) fluoro-4 benzamide
— N-(bromo-7 naphtyridine-1,8 yl-2) méthoxy-4 benzamide
— N-(méthoxy-7 naphtyridine-1,8 yl-2) méthoxy-4 benzamide
— N-(méthoxy-7 naphtyridine-1,8 yl-2) thiophènecarboxamide-2.

Les exemples suivants illustrent la présente invention:

*Exemple 1*

A une solution de 10,9 g d'acide p. toluique dans 100 $cm^3$ de tétrahydrofuranne anhydre, on ajoute 12,9 g de N,N'-carbonyldiimidazole. On observe un dégagement gazeux immédiat. Le mélange est agité pendant 1 heure à une température voisine de 20°C, jusqu'à la fin du dégagement gazeux. On ajoute ensuite 8,9 g d'amino-2 chloro-7 naphtyridine-1,8 et on chauffe à reflux pendant 20 heures. Le mélange est versé sur 1000 $cm^3$ d'eau distillée, le précipité formé est séparé par filtration, lavé à l'eau et séché à l'air.

Le produit obtenu (13,6 g; F = 222°C) est dissous dans 470 $cm^3$ d'éthanol bouillant. Après 4 heures de refroidissement à 4°C, le solide cristallisé est séparé par filtration, lavé par 3 fois 20 $cm^3$ d'éthanol et séché à 40°C sous pression réduite (0,067 kPa). On obtient 10 g de N-(chloro-7 naphtyridine-1,8 yl-2) méthyl-4 benzamide fondant à 228°C.

*Exemple 2*

On opère d'une manière analogue à celle décrite à l'exemple 1, mais à partir de 5,2 g d'acide cyclopropylcarboxylique, de 9,8 g de N,N'-carbonyldiimidazole et de 7,2 g d'amino-2 chloro-7 naphtyridine-1,8. Le produit obtenu par précipitation dans l'eau (10 g; F = 250°C) est dissous dans 530 $cm^3$ de propanol-2 bouillant.

Après 4 heures de refroidissement à 4°C le solide cristallisé est séparé par filtration, lavé par 3 fois 20 $cm^3$ de propanol-2 et séché à 40°C sous préssion réduite (0,067 kPa). On obtient 7,5 g de N-(chloro-7 naphtyridine-1,8 yl-2) cyclopropanecarboxamide fondant à 250°C.

*Exemple 3*

On opère d'une manière analogue à celle décrite à l'exemple 1, mais à partir de 13,7 g d'acide cyclopropanecarboxylique, de 25,8 g de N,N'-carbonyldiimidazole et de 17,5 g d'amino-2 méthoxy-7 naphtyridine-1,8. Le produit obtenu par précipitation dans l'eau (24,3 g; F = 100°C) est purifié par chromatographie sur une colonne de 40 mm de diamètre contenant 400 g de silice (0,040 - 0,063 mm) en éluant par un mélange (95-5 en volumes) de chlorure de méthylène et de méthanol et en recueillant des fractions de 100 $cm^3$. Après concentration à sec des fractions 6 à 17 à 40°C sous pression réduite (4 kPa), on obtient 17 g d'un solide fondant à 170°C. Ce produit est dissous dans 120 $cm^3$ d'éthanol bouillant. Après 2 heures de refroidissement à 4°C, le solide cristallisé est séparé par filtration, lavé par 2 fois 10 $cm^3$ d'éthanol et séché à 40°C sous pression réduite (0,067 kPa). On obtient 12,5 g de N-(méthoxy-7 naphtyridine-1,8 yl-2) cyclopropanecarboxamide fondant à 170°C.

L'amino-2 méthoxy-7 naphtyridine-1,8 peut être préparée selon la méthode décrite dans le brevet US 3 948 917.

*Exemple 4*

On opère d'une manière analogue à celle décrite à l'exemple 1, mais à partir de 4,8 g d'acide cyclopropane carboxylique, de 9,07 g de N,N'-carbonyldiimidazole et de 10 g d'amino-2 phénoxy-7 naphtyridine-1,8. Le produit obtenu par précipitation dans l'eau (11,2 g; F environ 170°C) est dissous dans 200 $cm^3$ d'éthanol bouillant. Après 2 heures de refroidissement à 4°C, le solide cristallisé est séparé par filtration, lavé par 2 fois 10 $cm^3$ d'éthanol et séché à 40°C sous pression réduite (0,067 kPa). On obtient 6,4 g de N-(phénoxy-7 naphtyridine-1,8 yl-2) cyclopropanecarboxamide fondant à 190°C.

L'amino-2 phénoxy-7 naphtyridine-1,8 peut être préparée de la façon suivante:

On chauffe pendant 20 heures à 120°C un mélange composé de 27 g d'amino-2 chloro-7

naphtyridine-1,8, de 141 g de phénol et de 19,8 g de potasse en pastilles à 85%. Le mélange obtenu est repris par 300 $cm^3$ d'une solution aqueuse de soude 4N et extrait par 250 $cm^3$ de chlorure de méthylène.

La phase aqueuse est extraite à nouveau par 2 fois 200 $cm^3$ de chlorure de méthylène. Les extraits organiques sont lavés par 2 fois 150 $cm^3$ de soude 4N puis 250 $cm^3$ d'eau distillée, séchés sur sulfate de magnésium et concentrés à sec à 40°C sous pression réduite (4 kPa).

Le produit obtenu (30,5 g; F = 190-194°C) est dissous dans 400 $cm^3$ d'acétonitrile bouillant. Après 2 heures de refroidissement à 4°C, le solide cristallisé est séparé par filtration, lavé par 20 $cm^3$ d'acétonitrile et séché à 45°C, sous pression réduite (0,067 kPa). On obtient 23,4 g d'amino-2 phénoxy-7 naphtyridine-1,8 fondant à 196°C.

*Exemple 5*

On opère d'une manière analogue à celle décrite à l'exemple 1, mais à partir de 5 g d'acide cyclobutane carboxylique, de 10,5 g de N,N'-carbonyldiimidazole et de 7 g d'amino-2 chloro-7 naphtyridine-1,8. Le produit obtenu par filtration (9 g; F = 192°C) est dissous dans 150 $cm^3$ d'acétonitrile bouillant. Après 3 heures de refroidissement à 20°C, le solide cristallisé est séparé par filtration, lavé par 3 fois 25 $cm^3$ d'éther éthylique et séché à 35°C sous pression réduite (0,066 kPa). On obtient 6,3 g de N-(chloro-7 naphtyridine-1,8 yl-2) cyclobutanecarboxamide fondant à 192-194°C.

*Exemple 6*

On opère d'une manière analogue à celle décrite à l'exemple 1, mais à partir de 6,2 g d'acide cyclohexadiène-1,4 carboxylique-1, de 8 g de N,N'-carbonyldiimidazole et de 8 g d'amino-2 chloro-7 naphtyridine-1,8. Le produit obtenu par précipitation dans l'eau (11 g; F = 205°C) est dissous dans 250 $cm^3$ d'éthanol bouillant. Après 2 heures de refroidissement à 4°C, le solide cristallisé est séparé par filtration, lavé par 3 fois 15 $cm^3$ d'éthanol et séché à 40°C sous pression réduite (0,067 kPa). On obtient 4,2 g de N-(chloro-7 naphtyridine-1,8 yl-2) cyclohexadiène-1,4 carboxamide-1 fondant à 210°C.

L'acide cyclohexadiène-1,4 carboxylique-1 peut être préparé selon la méthode décrite par A.A. Petrov. et coll., Zhur Obshchei Khim, 26, (1956) [C.A. 51 1887e (1957)].

*Exemple 7*

On opère d'une manière analogue à celle décrite à l'exemple 1, mais à partir de 12,4 g d'acide cyclohexadiène-1,4 carboxylique-1, de 16,2 g de N,N'-carbonyldiimidazole et de 21,4 g d'amino-2 phénoxy-7 naphtyridine-1,8. Le produit obtenu par précipitation dans l'eau (17,2 g; F = 95°C) est dissous dans 150 $cm^3$ de méthanol bouillant. Après 3 heures de refroidissement à 20°C, le solide cristallisé est séparé par filtration, lavé par 3 fois 15 $cm^3$ de méthanol et séché à 40°C sous pression réduite (0,067 kPa). On obtient 13,2 g de N-(phénoxy-7 naphtyridine-1,8 yl-2) cyclohexadiène-1,4 carboxamide-1 fondant à 95°C.

*Exemple 8*

A une solution de 5,4 g d'amino-2 chloro-7 naphthyridine-1,8 dans 60 $cm^3$ de pyridine, on ajoute lentement 4,2 g de chlorure de benzoyle en maintenant la température au voisinage de 30°C. Après avoir maintenu la solution pendant 3 heures à température voisine de 20°C, le produit est précipité dans 340 $cm^3$ d'eau distillée. Le solide obtenu après séchage à l'air est dissous dans 88 $cm^3$ d'éthanol bouillant. Après 3 heures de refroidissement à 4°C, le solide cristallisé est séparé par filtration, lavé par 2 fois 5 $cm^3$ d'éthanol et séché à 40°C sous pression réduite (0,067 kPa). On obtient ainsi 4,95 g de N-(chloro-7 naphtyridine-1,8 yl-2) benzamide fondant à 198°C.

*Exemple 9*

On opère d'une manière analogue à celle décrite à l'exemple 1, mais à partir de 13,4 g d'acide benzoïque, de 17,8 g de N,N'-carbonyldiimidazole et de 12,3 g d'amino-2 méthoxy-7 naphtyridine-1,8. Le produit obtenu par précipitation dans l'eau (20 g; P.F. = 80°C) est dissous dans 200 $cm^3$ d'acétonitrile bouillant. Après 1 heure de refroidissement à 4°C, le solide cristallisé est séparé par filtration, lavé par 2 fois 10 $cm^3$ d'acétonitrile et séché à 40°C sous pression réduite (0,067 kPa). On obtient 7,8 g de N-(méthoxy-7 naphtyridine-1,8 yl-2) benzamide fondant à 134°C.

*Exemple 10*

On opère d'une manière analogue à celle décrite à l'exemple 1, mais à partir de 8,9 g d'acide benzoïque de 11,8 g de N,N'-carbonyldiimidazole et de 15 g d'amino-2 benzylthio-7 naphtyridine-1,8. Le produit obtenu par précipitation dans l'eau (25 g; F environ 120°C) est purifié par chromatographie sur une colonne de 5 cm de diamètre contenant 600 g de silice (0,063 - 0,2 mm) en éluant par du chlorure de méthylène. On recueille des fractions de 100 $cm^3$, les fractions 14 à 56 sont concentrées à sec sous pression réduite (4 kPa) pour donner 22,4 g d'un solide fondant à 142 - 144°C. Ce produit est dissous dans 250 $cm^3$ d'éthanol bouillant. Après 2 heures de refroidissement à 4°C, le solide cristallisé est separé par filtration, lavé par 2 fois 25 $cm^3$ d'éthanol et séché à 40 sous pression réduite (0,067 kPa). On obtient 19 g de N-(benzylthio-7 naphtyridine-1,8 yl-2) benzamide fondant à 144-145°C.

L'amino-2 benzylthio-7 naphtyridine-1,8 peut être préparée de la façon suivante:

A une solution d'éthylate de sodium préparée à partir de 2,3 g de sodium et de 160 $cm^3$ d'éthanol, on ajoute 13,6 g de benzylmercaptan et on agite pendant 1 heure à une température voisine de 20°C. On ajoute ensuite 18 g d'amino-2 chloro-7 naphtyridine-1,8 et on chauffe pendant 6 heures à 60°C. La suspension obtenue est versée dans 500 $cm^3$ d'eau et extraite par 3 fois 250 $cm^3$ de chlorure de méthylène. Après lavage à l'eau et séchage la solution organique est concentrée à sec sous pression reduite (4 kPa) pour donner 15,4 g d'amino-2 benzylthio-7 naphtyridine-1,8 fondant à 167°C.

*Exemple 11*

A une solution de 6,8 g d'acide benzoïque dans 180 $cm^3$ de tétrahydrofuranne anhydre, on ajoute 9,1 g de N,N'-carbonyldiimidazole. On observe un dégagement gazeux immédiat. Le mélange est agité pendant 2 heures à une température voisine de 20°C, jusqu'à la fin du dégagement gazeux. On ajoute ensuite 10 g d'amino-2 phénoxy-7 naphtyridine-1,8 et on chauffe à reflux pendant 18 heures.

Le mélange est versé sur 800 $cm^3$ d'eau distillée; le précipité formé est séparé par filtration, lavé à l'eau et séché à l'air.

Le produit obtenu (14 g; F = 169°C) est dissous dans 160 $cm^3$ d'acétonitrile bouillant. Après 2 heures de refroidissement à 4°C, le solide cristallisé est séparé par filtration, lavé par 2 fois 8 $cm^3$ d'acétonitrile et séché à 25°C sous pression réduite (0,067 kPa). On obtient 8 g de N-(phénoxy-7 naphtyridine--1,8 yl-2) benzamide fondant à 170°C.

*Exemple 12*

On opère d'une manière analogue à celle décrite à l'exemple 1, mais à partir de 11 g d'acide benzoïque, de 16,2 g de N,N'-carbonyldiimidazole et de 26,7 g d'amino-2 (méthoxy-4 phénoxy)-7 naphtyridine-1,8. Le produit obtenu par précipitation dans l'eau (30,8 g; F environ 110°C) est dissous dans 150 $cm^3$ d'acétonitrile bouillant. Après 3 heures de refroidissement à 4°C, le solide cristallisé est séparé par filtration, lavé par 2 fois 5 $cm^3$ d'acétonitrile et séché à 50°C sous pression réduite (0,067 kPa). On obtient 24,7 g de N-[(méthoxy-4 phenoxy)-7 naphtyridine-1,8 yl-2] benzamide fondant à 171°C.

L'amino-2 (méthoxy-4 phénoxy)-7 naphthyridine-1,8 peut être préparée de la façon suivante:

On opère d'une manière analogue à celle décrite à l'exemple 4, mais à partir de 35,8 g d'amino-2 chloro-7 naphtyridine-1,8 de 99,2 g de méthoxy-4 phénol et de 22,4 g de potasse en pastilles à 85%. Après traitement à la soude et lavage, on obtient 52,9 g d'amino-2 (méthoxy-4 phénoxy)-7 naphtyridine-1,8 fondant à 200°C.

*Exemple 13*

On opère d'une manière analogue à celle décrite à l'exmple 1, mais à partir de 11,2 g d'acide fluoro-2 benzoïque, de 12,9 g de N,N'-carbonyldiimidazole et de 8,9 g d'amino-2 chloro-7 naphtyridine-1,8. Le produit obtenu par précipitation dans l'eau (13,6 g; F = 218°C) est dissous dans 450 $cm^3$ de propanol-1 bouillant. Après 3 heures de refroidissement à 4°C le solide cristallisé est séparé par filtration, lavé par 3 fois 20 $cm^3$ de propanol-1 et séché à 40°C sous pression réduite (0,067 kPa). On obtient 8,3 g de N-(chloro-7 naphtyridine-1,8 yl-2) fluoro-2 benzamide fondant à 222°C.

*Exemple 14*

On opère d'une manière analogue à celle décrite à l'exemple 1, mais à partir de 20 g d'acide fluoro-3 benzoïque, de 23 g de N,N'-carbonyldiimidazole et de 16,9 g d'amino-2 chloro-7 naphtyridine-1,8. Le produit obtenu par précipitation dans l'eau (25 g; F = 201°C) est dissous dans 230 $cm^3$ d'acétonitrile bouillant. Après 4 heures de refroidissement à 4°C le solide cristallisé est séparé par filtration, lavé par 2 fois 20 $cm^3$ d'acétonitrile et séché à 40°C sous pression réduite (0,067 kPa). On obtient 21,4 g de N-(chloro-7 naphtyridine-1,8 yl-2) fluoro-3 benzamide fondant à 202°C.

*Exemple 15*

On opère d'une manière analogue à celle décrite à l'exemple 1, mais à partir de 11,2 g d'acide fluoro-4 benzoïque, de 12,9 g de N,N'-carbonyldiimidazole et de 8,9 g d'amino-2 chloro-7 naphtyridine-1,8. Le produit obtenu par précipitation dans l'eau (14,3 g; F = 230°C) est dissous dans 800 $cm^3$ de propanol-2 bouillant. Après 4 heures de refroidissement à 4°C le solide cristallisé est séparé par filtration, lavé par 3 fois 10 $cm^3$ de propanol-2 et séché à 40°C sous pression réduite (0,067 kPa). On obtient 10,3 g de N-(chloro-7 naphtyridine-1,8 yl-2) fluoro-4 benzamide fondant à 236°C.

*Exemple 16*

On opère d'une manière analogue à celle décrite à l'exemple 1, mais à partir de 15,4 g d'acide fluoro-4 benzoïque, de 17,8 g de N,N'-carbonyldiimidazole et de 15,7 g d'amino-2 bromo-7 naphtyridine-1,8. Le produit obtenu par précipitation dans l'eau (26,2 g; F = 226°C) est purifié par filtration sur une colonne de 45 mm de diamètre contenant 300 g de silice (0,040 - 0,063 mm) en éluant par le dichlorométhane pur et en recueillant des fractions de 100 $cm^3$. Après concentration à sec des fractions 10 à 70 à 40°C sous pression réduite (4 kPa), on obtient 18,5 g d'un solide fondant à 228°C. Ce produit est dissous dans 600 $cm^3$ d'éthanol bouillant. Après 2 heures de refroidissement à 4°C, le solide cristallisé est séparé par filtration, lavé par 10 $cm^3$ d'éthanol et séché à 40°C sous pression réduite (0,067 kPa). On obtient 13,1 g de N-(bromo-7 naphtyridine-1,8 yl-2) fluoro-4 benzamide fondant à 228°C.

*Exemple 17*

On opère d'une manière analogue à celle décrite à l'exemple 1, mais à partir de 12,1 g d'acide difluoro-2,6 benzoïque, de 12,3 g de N-N'-carbonyldiimidazole et de 8,5 g d'amino-2 chloro-7 naphthyridine-1,8. Le produit obtenu par précipitation dans l'eau (6,8 g; F = 215°C) est dissous dans 450 $cm^3$ d'éthanol bouillant. Après 4 heures de refroidissement à 4°C, le solide cristallisé est séparé par filtration, lavé par 3 fois 15 $cm^3$ d'éthanol et séché à 40°C sous pression réduite (0,067 kPa). On obtient 7,3 g de N-(chloro-7 naphtyridine-1,8 yl-2) difluoro-2,6 benzamide fondant à 242°C.

*Exemple 18*

On opère d'une manière analogue à celle décrite à l'exemple 1, mais à partir de 5,1 g d'acide difluoro-2,6 benzoïque, de 5,2 g de N,N'-carbonyldiimidazole et de 4,4 g d'amino-2 méthoxy-7 naphtyridine-1,8. Le produit obtenu par précipitation dans l'eau (4,5 g; F = 215-217°C) est dissous dans 70 $cm^3$ d'éthanol bouillant. Après 1 heure de refroidissement à 4°C, le solide cristallisé est séparé par filtration, lavé par 10 $cm^3$ d'éthanol et séché à

à 45°C sous pression réduite (0,067 kPa). On obtient 3,2 g de N-(méthoxy-7 naphtyridine-1,8 yl-2 difluoro-2,6 benzamide fondant à 219-220°C.

*Exemple 19*

A une solution de 3,5 g d'amino-2 méthoxy-7 naphtyridine dans 40 $cm^3$ de pyridine, on ajoute 4,4 g de chlorure d'acétoxy-3 benzoyle. Le mélange est agité pendant 1 heure 30 minutes à une température voisine de 25°C. La suspension obtenue est reprise par 400 $cm^3$ d'eau distillée et le précipité obtenu est séché à l'air pour donner 6,6 g d'un solide fondant à 172°C. Ce produit est dissous dans 140 $cm^3$ d'éthanol bouillant. Après 3 heures de refroidissement à 4°C, le solide cristallisé est séparé par filtration, lavé par 3 fois 20 $cm^3$ d'éthanol et séché à 50°C sous pression réduite (0,067 kPa). On obtient 5,3 g de N-(méthoxy-7 naphtyridine-1,8 yl-2) acétoxy-3 benzamide fondant à 172°C. 12 g de N-(méthoxy-7 naphtyridine-1,8 yl-2) acétoxy-3 benzamide obtenus dans les conditions décrites ci-dessus sont traités par chauffage à reflux pendant 15 minutes dans 35 $cm^3$ d'une solution à 10% de potasse éthanolique. Le solide obtenu après dilution par 250 $cm^3$ d'eau et acidifiée par 20 $cm^3$ d'acide chlorhydrique 4N est séché à l'air pour donner 9,2 g d'un solide fondant à une température supérieure à 260°C. Le produit obtenu est dissous dans 1500 $cm^3$ de propanol-1 bouillant. Après reforidissement pendant 4 heures à une température de 4°C, le solide cristallisé est séparé par filtration, lavé par 3 fois 50 $cm^3$ de propanol-1 et séché à 60°C sous pression réduite (0,067 kPa). On obtient 6 g de N-(méthoxy-7 naphtyridine-1,8 yl-2) hydroxy-3 benzamide fondant à 295°C.

*Exemple 20*

On opère d'une manière analogue à celle décrite à l'exemple 1, mais à partir de 4,55 g d'acide méthoxy-4 benzoïque, de 6,55 g de N,N'-carbodiimidazole et de 5,2 g d'amino-2 naphthyridine-1,8. Le produit obtenu par précipitation dans l'eau (8,35 g; F = 85°C, pateux) est filtré, puis dissous dans 50 $cm^3$ de propanol-1 bouillant. Après 1 heure de refroidissement à 4°C le solide cristallisé est séparé par filtration, lavé par 2 fois 5 $cm^3$ de propanol-1 et séché à 35°C sous pression réduite (0,067 kPa). On obtient 6,9 g de N-(naphtyridine-1,8 yl-2) méthoxy-4 benzamide fondant à 150°C.

L'amino-2 naphtyridine-1,8 peut être préparée selon le brevet US 3 948 917.

*Exemple 21*

On opère d'une manière analogue à celle décrite à l'exemple 1, mais à partir de 12,17 g d'acide méthoxy-3 benzoïque, 12,9 g de N,N'-carbonyldiimidazole et de 8,9 g d'amino-2 chloro-7 naphtyridine-1,8. Le produit obtenu par précipitation dans l'eau (15,5 g; F = 176°C) est dissous dans 500 $cm^3$ de propanol-2 bouillant. Après 2 heures de refroidissement à 4°C, le solide cristallisé est séparé par filtration, lavé par 3 fois 25 $cm^3$ de propanol-2 et séché à 40°C sous pression réduite (0,067 kPa). On obtient 11,2 g de N-(chloro-7 naphtyridine-1,8 yl-2) méthoxy-3 benzamide fondant à 178°C.

*Exemple 22*

On opère d'une manière analogue à celle décrite à l'exemple 1, mais à partir de 4,6 g d'acide méthoxy-4 benzoïque, de 4,9 g de N,N'-carbonyldiimidazole et de 3,6 g d'amino-2 chloro-7 naphtyridine-1,8. Le produit obtenu par précipitation dans l'eau (4,3 g; F = 208°C) est dissous dans 660 $cm^3$ d'acétonitrile bouillant. Après 3 heures de refroidissement à 4°C, le solide cristallisé est séparé par filtration, lavé par 3 fois 20 $cm^3$ d'acétonitrile et séché à 50°C sous pression réduite (0,067 kPa). On obtient 3,5 g de N-(chloro-7 naphthyridine-1,8 yl-2) méthoxy-4 benzamide fondant à 208°C.

*Exemple 23*

On opère d'une manière analogue à celle décrite à l'exemple 1, mais à partir de 10,2 g d'acide méthoxy-4 benzoïque, de 10,8 g de N,N'-carbonyldiimidazole et de 11,2 g d'amino-2 bromo-7 naphtyridine-1,8. Le produit obtenu par précipitation dans l'eau (9,8 g; F = 220°C) est dissous dans 1000 $cm^3$ d'acétonitrile bouillant. Après 2 heures de refroidissement à 4°C le solide cristallisé est séparé par filtration, lavé par 2 fois 10 $cm^3$ d'acétonitrile et séché à 40°C sous pression réduite (0,067 kPa). On obtient 8,3 g de N-(bromo-7 naphtyridine-1,8 yl-2) méthoxy-4 benzamide fondant à 221°C.

L'amino-2 bromo-7 naphtyridine-1,8 peut être préparée selon la méthode décrite par S. CARBONI, Gazz. Chim. Ital. 96, 1464 (1966).

*Exemple 24*

On opère d'une manière analogue à celle décrite à l'exemple 1, mais à partir de 12,2 g d'acide méthoxy-4 benzoïque, de 10,3 g de N,N'-carbonyldiimidazole et de 9,5 g d'amino-2 méthyl-7 naphtyridine-1,8. Le produit obtenu par précipitation dans l'eau (14,4 g; F = 110°C) est dissous dans 150 $cm^3$ d'acétonitrile bouillant. Après 2 heures de refroidissement à 4°C, le solide cristallisé est séparé par filtration, lavé par 2 fois 10 $cm^3$ d'acétonitrile et séché à 45°C sous pression réduite (0,067 kPa). On obtient 12,7 g de N-(méthyl-7 naphtyridine-1,8 yl-2) méthoxy-4 benzamide fondant à 119°C.

L'amino-2 méthyl-7 naphtyridine-1,8 peut être préparée selon la méthode décrite par E.V. BROWN, J. Org. Chem. 30, 1607 (1965).

*Exemple 25*

On opère d'une manière analogue à celle décrite à l'exemple 1, mais à partir de 9,1 g d'acide méthoxy-3 benzoïque de 9,7 g de N,N'-carbonyldiimidazole et de 7 g d'amino-2 méthoxy-7 naphtyridine-1,8. Le produit obtenu par précipitation dans l'eau (13 g; F environ 75°C) est dissous dans 90 $cm^3$ d'acétonitrile bouillant. Après 3 heures de refroidissement à 4°C le solide cristallisé est séparé par filtration, lavé par 3 fois 10 $cm^3$ d'acétonitrile et séché à 50°C sous pression réduite (0,067 kPa). On obtient 8,7 g de N-(méthoxy-7 naphtyridine-1,8 yl-2) méthoxy-3 benzamide fondant à 135°C.

*Exemple 26*

On opère d'une manière analogue à celle décrite à l'exemple 1, mais à partir de 13,2 g d'acide

méthoxy-4 benzoïque, de 14,1 g de N,N'-carbonyldiimidazole et de 11,5 g d'amino-2 méthoxy-7 naphtyridine-1,8. Le produit obtenu par précipitation dans l'eau est séché sous pression réduite (0,067 kPa) et purifié par filtration sur une colonne de 5 cm de diamètre chargée avec 400 g de silice (0,063-0,2 mm) en éluant avec du chlorure de méthylène.

On recueille des fractions de 100 $cm^3$. Les fractions 8 à 38 sont réunies et concentrées à sec sous pression réduite (4 kPa). Le produit obtenu est dissous dans 150 $cm^3$ d'acétonitrile bouillant. Après 4 heures de refroidissement à 4°C, le solide cristallisé est séparé par filtration, lavé par 2 fois 10 $cm^3$ d'acétonitrile et séché à 40°C sous pression réduite (0,067 kPa).

On obtient 10,9 g de N-(méthoxy-7 naphtyridine-1,8 yl-2) méthoxy-4 benzamide fondant à 115°C.

L'amino-2 méthoxy-7 naphtyridine-1,8 peut être préparée selon le brevet US 3 948 917.

*Exemple 27*

On opère d'une manière analogue à celle décrite à l'exemple 1, mais à partir de 19,8 g d'acide méthoxy-4 benzoïque, de 21,1 g de N,N'-carbonyldiimidazole et de 18,5 g d'amino-2 éthoxy-7 naphtyridine-1,8. Le produit obtenu par précipitation dans l'eau (28,9 g; F = 144°C) est dissous dans 280 $cm^3$ d'éthanol bouillant. Après 3 heures de refroidissement à 4°C, le solide cristallisé est séparé par filtration, lavé par 2 fois 10 $cm^3$ d'éthanol et séché à 40°C sous pression réduite (0,067 kPa). On obtient 24 g de N-(éthoxy-7 naphtyridine-1,8 yl-2) méthoxy-4 benzamide fondant à 152°C.

L'amino-2 éthoxy-7 naphtyridine-1,8 peut être préparée selon la méthode décrite par S. CARBONI, Gazz. Chim. Ital. 96, 1456 (1966).

*Exemple 28*

On opère d'une manière analogue à celle décrite à l'exemple 1, mais à partir de 16,7 g d'acide méthoxy-4 benzoïque, de 19,4 g de N,N'-carbonyldiimidazole et de 25 g d'amino-2 isopropoxy-7 naphtyridine-1,8. Le mélange réactionnel est versé dans l'eau et extrait à l'acétate d'éthyle. Après concentration à sec sous pression réduite (4 kPa) l'huile obtenue (39 g) est agitée en présence de 100 $cm^3$ d'oxyde de diéthyle pour donner un solide cristallisé (23 g; F = 78°C). Le produit obtenu est dissous dans 100 $cm^3$ d'oxyde d'isopropyle bouillant. Après 2 heures de refroidissement à 4°C le solide cristallisé est séparé par filtration, lavé par 2 fois 10 $cm^3$ d'oxyde de diéthyle et séché à 40°C sous pression réduite (0,067 kPa). On obtient 12,9 g de N-(isopropoxy-7 naphtyridine-1,8 yl-2) méthoxy-4 benzamide fondant à 82°C.

L'amino-2 isoporpoxy-7 naphtyridine-1,8 peut être préparée de la façon suivante:

On opère d'une manière analogue à celle décrite à l'exemple 29, mais à partir de 35,9 g d'amino-2 chloro-7 naphtyridine-1,8 de 150 $cm^3$ de propanol-2 et de 9,2 g de sodium. Le propanol-2 en excès est évaporé sous pression réduite (4 kPa) et le résidu est repris par de l'eau distillée pour conduire à un solide cristallisé (38,7 g; F = 162°C). 11 g du solide obtenu sont dissous dans 50 $cm^3$ d'acétate d'éthyle bouillant. Après 2 heures de refroidissement à 4°C le solide cristallisé est séparé par filtration, lavé par 2 fois 5 $cm^3$ d'oxyde de diéthyle et séché à 50°C sous pression réduite (0,067 kPa). On obtient 6,9 g d'amino-2 isopropoxy-7 naphtyridine-1,8 fondant à 170°C.

*Exemple 29*

On opère d'une manière analogue à celle décrite à l'exemple 1, mais à partir de 5,8 g d'acide méthoxy-4 benzoïque, de 6,5 g de N,N'-carbonyldiimidazole et de 8,5 g d'amino-2 (méthoxy-2 éthoxy)-7 naphtyridine-1,8. Le produit obtenu par précipitation dans l'eau (8 g; F = pâteux 75°C) est putifié par chromatographie sur une colonne de 40 mm de diamètre contenant 250 g de silice (0,063 - 0,2 mm) en éluant par un mélange (99,5 - 0,5 en volumes) de chlorure de méthylène et de méthanol. On recueille des fractions de 100 $cm^3$, les fractions 12 à 31 sont réunies et concentrées à sec sous pression réduite (4 kPa) à 40°C pour donner 10,3 g d'un solide fondant à 114°C. Ce produit est dissous dans 50 $cm^3$ de tétrachlorure de carbone bouillant. Après 16 heures de refroidissement à 4°C, le solide cristallisé est séparé par filtration, lavé par 2 fois 5 $cm^3$ de tétrachlorure de carbone et séché à 40°C sous pression réduite (0,067 kPa). On obtient 8,5 g de N-[(méthoxy-2 éthoxy)-7 naphtyridine-1,8 yl-2) méthoxy-4 benzamide] fondant à 114°C.

L'amino-2 (méthoxy-2 éthoxy)-7 naphtyridine-1,8 peut être préparée de la façon suivante:

L'alcoolate de méthoxy-2 éthanol est préparé à partir de 100 $cm^3$ d'alcool correspondant et de 9,2 g de sodium. Lorsque le dégagement gazeux a cessé, 35,9 g d'amino-2 chloro-7, naphtyridine-1,8 sont ajoutés et le mélange réactionnel est agité à 120°C pendant 2 heures 30 minutes puis versé après refroidissement dans 100 $cm^3$ d'eau. Le solide obtenu est filtré et séché à l'air (30 g; F = 175°C). 10 g de produit obtenu sont dissous dans 175 $cm^3$ de propanol-2 bouillant. Après 3 heures de refroidissement à 4°C, le solide cristallisé est séparé par filtration, lavé par 10 $cm^3$ de propanol-2 et séché à 50°C sous pression réduite (0,067 kPa). On obtient 8,9 g d'amino-2 (méthoxy-2 éthoxy)-7 naphtyridine-1,8 fondant à 176°C.

*Exemple 30*

On opère d'une manière analogue à celle décrite à l'exemple 1, mais à partir de 9,6 g d'acide méthoxy-4 benzoïque de 10,2 g de N,N'-carbonyldiimidazole et de 8,5 g d'allyloxy-2 amino-7 naphtyridine-1,8. Le produit obtenu par précipitation dans l'eau (13,9 g; F environ 70°C) est purifié par chromatographie sur une colonne de 40 mm de diamètre contenant 250 g de silice (0,040 - 0,063 mm) en éluant par un mélange (99-1 en volumes) de chlorure de méthylène et de méthanol. On recueille des fractions de 100 $cm^3$, les fractions 9 à 23 sont réunis et concentrées à sec à 40°C sous pression réduite (4 kPa) pour donner 10 g d'un solide gras. Ce produit est repris dans 50 $cm^3$ d'oxyde d'isopropyle. Après 30 minutes d'agitation à 20°C, le solide cristallisé est séparé

par filtration, lavé par 2 fois 10 $cm^3$ d'oxyde d'isopropyle et séché à 30°C sous pression réduite (0,067 kPa). On obtient 8,9 g de N-(allyloxy-7 naphtyridine-1,8 yl-2) méthoxy-4 benzamide fondant à 108°C.

L'allyloxy-2 amino-7 naphtyridine-1,8 peut être préparé de la façon suivante:

On opère d'une manière analogue à celle décrite à l'exemple 29, mais à partir de 17,9 g d'amino-2 chloro-7 naphtyridine-1,8 de 75 $cm^3$ d'alcool allylique et de 4,6 g de sodium.

Après avoir versé le mélange réactionnel dans l'eau, on obtient 17,6 g d'un solide cristallisé fondant à 138°C. 7 g du produit obtenu sont dissous dans 210 $cm^3$ de tétrachlorure de carbone bouillant. Après 3 heures de refroidissement à 4°C, le solide cristallisé est séparé par filtration, lavé par 3 fois 10 $cm^3$ de tétrachlorure de carbone et séché à 50°C sous pression réduite (0,067 kPa). On obtient 6,4 g d'allyloxy-2 amino-7 naphtyridine-1,8 fondant à 138°C.

*Exemple 31*

On opère d'une manière analogue à celle décrite à l'exemple 1, mais à partir de 11,5 g d'acide méthoxy-4 benzoïque, de 12 g de N,N'-carbonyldiimidazole et de 10 g d'amino-2 propargyloxy-7 naphtyridine-1,8. Le produit obtenu par précipitation dans l'eau (15,3 g; F = 140°C) est dissous dans 190 $cm^3$ d'éthanol bouillant. Après 3 heures de refroidissement à 4°C le solide cristallisé est séparé par filtration, lavé par 3 fois 20 $cm^3$ d'éthanol et séché à 50°C sous pression réduite (0,067 kPa). On obtient 10,1 g de N-(propargyloxy-7 naphtyridine-1,8 yl-2) méthoxy-4 benzamide fondant à 140°C.

L'amino-2 propargyloxy-7 naphtyridine-1,8 peut être préparée de la façon suivante:

On opère d'une manière analogue à celle décrite à l'exemple 29, mais à partir de 179 g d'amino-2 chloro-7 naphthyridine-1,8 de 750 $cm^3$ d'alcool propargylique et de 46 g de sodium. Après précipitation dans l'eau, la gomme obtenue (120 g) est reprise sous agitation par 500 $cm^3$ d'oxyde d'isopropyle. Le solide ainsi obtenu (114,5 g; F environ 150°C) est purifié par chromatographie sur une colonne de 50 mm de diamètre contenant 1 kg de silice (0,063 - 0,2 mm) en éluant par du chlorure de méthylène et en recueillant des fractions de 100 $cm^3$ (fractions 11 à 20) puis de 500 $cm^3$ (fractions 21 à 26). Après concentrations à sec des fractions 6 à 26 sous pression réduite (4 kPa), le résidu obtenu est lavé sous agitation à l'éther éthylique, filtré puis séché. On obtient 21 g d'un solide fondant à 184°C. 10,8 g de ce produit sont dissous dans 100 $cm^3$ d'acétonitrile bouillant. Après 3 heures de refroidissement à 4°C, le solide cristallisé est séparé par filtration, lavé par 3 fois 20 $cm^3$ d'acétonitrile et séché à 40°C sous pression réduite (0,067 kPa). On obtient 6,8 g d'amino-2 propargyloxy-7 naphtyridine-1,8.

*Exemple 32*

On opère d'une manière analogue à celle décrite à l'exemple 1, mais à partir de 11,8 g d'acide méthoxy-4 benzoïque, de 12,6 g de N,N'-carbonyldiimidazole et de 9,6 g d'amino-2 méthylthio-7 naphthyridine-1,8. Le produit obtenu par précipitation dans l'eau (15,8 g; F = 100°C) est dissous dans 180 $cm^3$ de propanol-2 bouillant. Après 1 heure de refroidissement à 4°C, le solide cristallisé est séparé par filtration, lavé par 10 $cm^3$ de propanol-2 puis par 2 fois 10 $cm^3$ d'oxyde d'isopropyle et séché à 40°C sous pression réduite (0,067 kPa). On obtient 13,6 g de N-(méthylthio-7 naphtyridine-1,8 yl-2) méthoxy-4 benzamide fondant à 130°C.

La méthylthio-1 amino-7 naphtyridine-1,8 peut être préparée de la façon suivante:

A une solution, proche de la saturation, de méthylmercaptan dans 200 $cm^3$ de diméthylformamide maintenu à 0°C, on additionne, par petites portions 13,3 g d'hydrure de sodium en suspension à 50% dans l'huile minérale. On ajoute ensuite à cette solution, 25 g d'amino-2 chloro-7 naphtyridine-1,8 et on chauffe le mélange à 100°C pendant 2 heures. Après refroidissement le mélange réactionnel est versé dans 500 $cm^3$ d'eau et on extrait avec 5 fois 250 $cm^3$ de chlorure de méthylène. Les extraits organiques sont ensuite lavés avec 2 fois 200 $cm^3$ d'eau distillée, séchés sur sulfate de magnésium et concentrés à sec à 40°C sous pression réduite (4 kPa). Le produit obtenu (22,9 g; F = 130°C) est dissous dans 150 $cm^3$ d'éthanol bouillant. Après 2 heures de refroidissement à 4°C, le solide cristallisé est séparé par filtration, lavé par 10 $cm^3$ d'éthanol puis par 3 fois 10 $cm^3$ d'oxyde d'isopropyle et séché à 40°C sous pression réduite (0,067 kPa). On obtient 9,6 g d'amino-2 méthylthio-7 naphtyridine-1,8 fondant à 158°C.

*Exemple 33*

En opérant d'une manière analogue à celle décrite à l'exemple 1, mais à partir de 4,1 g d'acide méthoxy-4 benzoïque de 4,4 g de N,N'-carbonyldiimidazole et de 4,75 g d'amino-2 phénoxy-7 naphtyridine-1,8. Le produit obtenu par précipitation dans l'eau (8,8 g) est purifié par chromatographie sur une colonne de 3 cm de diamètre contenant 140 g de silice (0,063 - 0,2 mm), en éluant par un mélange (99-1 en volumes) de chlorure de méthylène et d'acétate d'éthyle. On recueille des fractions de 50 $cm^3$, les fractions 7 à 20 sont réunies et concentrées à sec à 40°C sous pression réduite (4 kPa) pour donner 5,9 g d'un solide fondant à environ 150°C. Ce produit est dissous dans 70 $cm^3$ d'éthanol bouillant. Après 2 heures de refroidissement à 4°C, le solide cristallisé est séparé par filtration, lavé par 2 fois 10 $cm^3$ d'éthanol et séché à 40°C sous pression réduite (0,067 kPa). On obtient 10 g de N-(phénoxy-7 naphtyridine-1,8 yl-2) méthoxy-4 benzamide fondant à 160°C.

*Exemple 34*

On opère d'une manière analogue à celle décrite à l'exemple 1, mais à partir de 6,1 g d'acide méthoxy-4 benzoïque, de 6,5 g de N,N'-carbonyldiimidazole et de 7,65 g d'amino-2 (fluoro-2 phenoxy)-7 naphtyridine-1,8. Le produit obtenu par précipitation dans l'eau (5,5 g; F = 200°C) est dissous dans 180 $cm^3$ d'éthanol bouillant. Après 2 heures de refroidissement à 4°C, le solide cristallisé est séparé par filtration, lavé par 2 fois 10 $cm^3$ d'éthanol et séché à 40°C sous pression réduite (0,067 kPa). On obtient 3,8 g de N-[(fluoro-2 phénoxy)-7 naphtyridine-1,8 yl-2] méthoxy-4 benzamide fondant à 208°C.

L'amino-2 (fluoro-2 phénoxy)-7 naphtydine-1,8 peut être préparée de la façon suivante:

On opère d'une manière analogue à celle décrite à l'exemple 37, mais à partir de 18 g d'amino-2 chloro-7 naphtyridine-1,8 de 44,8 g de fluoro-2 phénol et de 13,2 g de potasse en pastilles à 85%. Après traitement par la soude et lavage, le produit obtenu (24 g; F = 202°C) est dissous dans 200 $cm^3$ d'éthanol bouillant. Après 16 heures de refroidissement à 4°C, le solide cristallisé est séparé par filtration, lavé par 2 fois 15 $cm^3$ d'éthanol et séché à 40°C sous pression réduite (0,067 kPa). On obtient 14,9 g d'amino-2 (fluoro-2 phénoxy)-naphtyridine-1,8 fondant à 206°C.

*Exemple 35*

On opère d'une manière analogue à celle décrite à l'exemple 1, mais à partir de 6,1 g d'acide méthoxy-4 benzoïque, de 6,5 g de N,N'-carbonyldiimidazole et de 7,65 g d'amino-2 (fluoro-3 phénoxy)-7 naphtyridine-1,8. Le produit obtenu par précipitation dans l'eau (14 g) est dissous dans 110 $cm^3$ d'éthanol bouillant. Après 2 heures de refroidissement à 4°C, le solide cristallisé est séparé par filtration, lavé par 2 fois 10 $cm^3$ d'éthanol et séché à 45°C sous pression réduite (0,067 kPa). On obtient 7 g ge N-[(fluoro-3 phénoxy)-7 naphtyridine-1,8 yl-2] méthoxy-4 benzamide fondant à 151°C.

L'amino-2 (fluoro-3 phénoxy)-7 naphtyridine-1,8 peut être préparée de la façon suivante:

On opère d'une manière analogue à celle décrite à l'exemple 37, mais à partir de 18 g d'amino-2 chloro-7 naphtyridine-1,8, de 44,8 g de fluoro-3 phénol et de 13,2 g de potasse en pastilles à 85%. Après traitement par la soude et lavage, le produit obtenu (23,5 g; F = 161°C) est dissous dans 120 $cm^3$ d'acétonitrile bouillant. Après 2 heures de refroidissement à 4°C, le solide cristallisé est séparé par filtration, lavé par 2 fois 10 $cm^3$ d'acétonitrile et séché à 40°C sous pression réduite (0,067 kPa). On obtient 16,5 g d'amino-2 (fluoro-3 phénoxy)-7 naphtyridine-1,8 fondant à 165°C.

*Exemple 36*

On opère d'une manière analogue à celle décrite à l'exemple 1, mais à partir de 14,3 g d'acide méthoxy-4 benzoïque, de 15,2 g de N,N'-carbonyldiimidazole et de 15,2 g d'amino-2 (fluoro-4 phénoxy)-7 naphtyridine-1,8. Le produit obtenu par précipitation dans l'eau (22,6 g; F environ 70°C) est dissous dans 130 $cm^3$ d'éthanol bouillant. Après 3 heures de refroidissement à 4°C, le solide cristallisé est séparé par filtration, lavé par 2 fois 15 $cm^3$ d'éthanol et séché à 25°C sous pression réduite (0,067 kPa). On obtient 17 g de N-[(fluoro-4 phénoxy)-7 naphtyridine-1,8 yl-2] méthoxy-4 benzamide fondant à 100°C.

L'amino-2 (fluoro-4 phénoxy)-7 naphtyridine-1,8 peut être préparée de la façon suivante:

On opère d'une manière analogue à celle décrite à l'exemple 4, mais à partir de 17,9 g d'amino-2 chloro-7 naphtyridine-1,8, de 44,8 g de fluoro-4 phénol et de 13,2 g de potasse en pastilles à 85%. Après 2 heures de chauffage à 115°C et traitement comme décrit précédemment à l'exemple 4, on obtient 15,2 g d'amino-2 (fluoro-4 phénoxy)-7 naphtyridine-1,8 fondant à 210°C.

*Exemple 37*

On opère d'une manière analogue à celle décrite à l'exemple 1, mais à partir de 16,4 g d'acide méthoxy-4 benzoïque, de 17,5 g de N,N'-carbonyldiimidazole et de 18,5 g d'amino-2 (chloro-2 phénoxy)-7 naphthyridine-1,8. Le produit obtenu par précipitation dans l'eau (28 g; F environ 80°C) est dissous dans 800 $cm^3$ d'éthanol bouillant. Après 15 heures de refroidissement à 4°C, le solide cristallisé est séparé par filtration, lavé par 2 fois 20 $cm^3$ d'éthanol et séché à 40°C sous pression réduite (0,067 kPa). On obtient 17,8 g de N-[(chloro-2 phénoxy)-7 naphtyridine-1,8 yl-2] méthoxy-4 benzamide fondant à 190°C.

L'amino-2 (chloro-2 phénoxy)-7 naphtyridine-1,8 peut être préparée de la façon suivante:

On chauffe pendant 4 heures à 120°C un mélange composé de 17,9 g d'amino-3 chloro-7 naphtyridine-1,8, de 51,4 g de chloro-2 phénol et 13,2 g de potasse en pastilles à 85%. Le mélange obtenu est versé dans 100 $cm^3$ de soude 4N, le précipité formé est séparé par filtration et lavé à l'eau jusqu'à pH = 7. Après séchage à 40°C sous pression réduite (0,067 kPa), on obtient 20,6 g d'amino-2 (chloro-2 phénoxy)-7 naphtyridine-1,8 fondant à 166°C.

*Exemple 38*

On opère d'une manière analogue à celle décrite à l'exemple 1, mais à partir de 9,7 g d'acide méthoxy-4 benzoïque, de 10,4 g de N,N'-carbonyldiimidazole et de 12,4 g d'amino-2 (bromo-2 phénoxy)-7 naphtyridine-1,8. Le produit obtenu par précipitation dans l'eau (17,6 g; F environ 70°C) est purifié par chromatographie sur une colonne de 45 mm de diamètre contenant 250 g de silice (0,040 - 0,063 mm) en éluant par un mélange (99-1 en volumes) de chlorure de méthylène et de méthanol et en recueillant des fractions de 50 $cm^3$. Après concentration à sec des fractions 5 à 17 à 40°C sous pression réduite (4 kPa), on obtient 10,4 g d'un solide fondant à environ 70°C. Ce produit est dissous dans 270 $cm^3$ d'éthanol bouillant. Après 3 heures de refroidissement à 4°C, le solide cristallisé est séparé par filtration, lavé par 2 fois 15 $cm^3$ d'éthanol et séché à 40°C sous pression réduite (0,067 kPa). On obtient 8,8 g de N-[(bromo-2 phénoxy)-7 naphtyridine-1,8 yl-2) méthoxy-4 benzamide fondant à 183°C.

L'amino-2 (bromo-2 phénoxy)-7 naphtyridine-1,8 peut être préparé de la façon suivante:

On opère d'une manière analogue à celle décrite à l'exemple 4, mais à partir de 19,7 g d'amino-2 chloro-7 naphtyridine-1,8 de 81,5 g de bromo-2 phénol et de 15,15 g de potasse en pastilles à 85%. Après 10 heures de chauffage à 120°C et traitement dans les conditions décrites précédemment à l'exemple 4, le produit obtenu (21,5 g; F = 160°C) est purifié par chromatographie sur une colonne

de 45 mm de diamètre contenant 300 g de silice (0,040 - 0,063 mm) en éluant par un mélange (95-5 en volumes) de chlorure de méthylène et de méthanol et en recueillant des fractions de 100 $cm^3$. Après concentration à sec des fractions 2 à 10 à 40°C sous pression réduite (4 kPa), on obtient 12,8 g d'amino-2 (bromo-2 phénoxy)-7 naphtyridine-1,8 fondant à 206°C.

*Exemple 39*

On opère d'une manière analogue à celle décrite à l'exemple 1, mais à partir de 15 g d'acide méthoxy-4 benzoïque, de 16 g de N,N'-carbonyldiimidazole et de 15,6 g d'amino-2 (méthyl-2 phénoxy)-7 naphtyridine-1,8. Le produit obtenu par précipitation dans l'eau (19 g; F = 50°C) est purifié par chromatographie sur une colonne de 45 mm de diamètre contenant 270 g de silice (0,040 - 0,063 mm) en éluant par le dichlorométhane pur. On recueille des fractions de 100 $cm^3$. Après concentration à sec des fractions 3 à 40 sous pression réduite (4 kPa) à 40°C, on obtient 14 g d'un solide fondant à environ 50°C. Ce produit est dissous dans 120 $cm^3$ d'éthanol bouillant. Après 4 heures de refroidissement à 4°C, le solide cristallisé est séparé par filtration, lavé par 10 $cm^3$ d'éthanol et séché à 40°C sous pression réduite (0,067 kPa). On obtient 11,1 g de N-[(méthyl-2 phénoxy)-7-naphtyridine-1,8 yl-2] méthoxy-4 benzamide fondant à 153°C.

L'amino-2 (méthyl-2 phénoxy)-7 naphtyridine-1,8 peut être préparée de la façon suivante:

On opère d'une manière analogue à celle décrite à l'exemple 4, mais à partir de 17,95 g d'amino-2 chloro-7 naphtyridine-1,8, de 43,2 g de méthyl-2 phénol et de 13,2 g de potasse en pastilles à 85%. Après traitement à la soude et lavage, le produit obtenu (21,2 g; F = 188°C) est dissous dans 100 $cm^3$ d'acétonitrile bouillant. Après 4 heures de refroidissement à 4°C, le solide cristallisé est séparé par filtration, lavé par 10 $cm^3$ d'acétonitrile et séché à 40°C sous pression réduite (0,067 kPa). On obtient 15,6 g d'amino-2 (méthyl-2 phénoxy)-7 naphtyridine-1,8 fondant à 192°C.

*Exemple 40*

On opère d'une manière analogue à celle décrite à l'exemple 1, mais à partir de 8,5 g d'acide méthoxy-4 benzoïque, de 9 g de N,N'-carbonyldiimidazole et de 9 g d'amino-2 (méthyl-3 phénoxy)-7 naphthyridine-1,8. Le produit obtenu par précipitation dans l'eau (10 g; F = 110°C) est purifié par filtration sur une colonne de 45 mm de diamètre contenant 150 g de silice (0,040 - 0,063 mm) en éluant par le dichlorométhane pur et en recueillant des fractions de 100 $cm^3$. Après concentration à sec des fractions 1 à 30 à 40°C sous pression réduite (4 kPa), on obtient 9 g d'un solide fondant à 158°C. Ce produit est dissous dans 120 $cm^3$ d'éthanol bouillant. Après 4 heures de refroidissement à 4°C, le solide cristallisé est séparé par filtration, lavé par 10 $cm^3$ d'éthanol et séché à 40°C sous pression réduite (0,067 kPa). On obtient 7,2 g de N-[(méthyl-3 phénoxy)-7 naphtyridine-1,8 yl-2] méthoxy-4 benzamide fondant à 160°C.

L'amino-2 (méthyl-3 phénoxy)-7 naphtyridine-1,8 peut être préparée de la façon suivante:

On opère d'une manière analogue à celle décrite à l'exemple 4, mais à partir de 17,95 g d'amino-2 chloro-7 naphtyridine-1,8 de 43,2 g de méthyl-3 phénol et de 13,2 g de potasse en pastilles à 85%. Après traitement par la soude et lavage, le produit obtenu (21,6 g; F = 148°C) est dissous dans 200 $cm^3$ d'acétate d'éthyle bouillant. Après 18 heures de refroidissement à 4°C, le solide cristallisé est séparé par filtration, lavé par 10 $cm^3$ d'acétate d'éthyle et séché à 40°C sous pression réduite (0,067 kPa). On obtient 9 g d'amino-2 (méthyl-3 phénoxy)-7 naphtyridine-1,8 fondant à 152°C.

*Exemple 41*

On opère d'une manière analogue à celle décrite à l'exemple 1, mais à partir de 16,7 g d'acide méthoxy-4 benzoïque, de 17,8 g de N,N'-carbonyldiimidazole et de 21,2 g d'amino-2 (diméthyl-2,6 phénoxy)-7 naphtyridine-1,8. Le mélange réactionnel est versé dans l'eau et extrait au chlorure de méthylène. Après concentration des phases organiques à sec sous pression réduite (4 kPa), le résidu solide (33 g) est dissous dans 170 $cm^3$ d'acétone bouillant. Après 2 heures de refroidissement à 4°C, le solide cristallisé est séparé par filtration, lavé par 10 $cm^3$ d'acétone et séché à 40°C sous pression réduite (0,067 kPa). Par recristallisation du solide obtenu (11,5 g; F environ 100°C) dans 50 $cm^3$ d'acétone on obtient 9,1 g de N-[(diméthyl-2,6 phénoxy)-7 naphtyridine-1,8 yl-2) méthoxy-4 benzamide fondant à 174°C.

L'amino-2 (diméthyl-2,6 phénoxy)-7 naphthyridine-1,8 peut être préparée de la façon suivante:

On opère d'une manière analogue à celle décrite à l'exemple 4, mais à partir de 17,95 g d'amino-2 chloro-7 naphtyridine-1,8, de 48,6 g de diméthyl-2,6 phénol et de 13,2 g de potasse en pastilles à 85%. Après traitement par la soude et lavage, le produit obtenu (24,8 g; F = 204°C) est purifié par chromatographie sur une colonne de 45 mm de diamètre, contenant 360 g de silice (0,040 - 0,063 mm) en éluant par du chlorure de méthylène pur et en recueillant des fractions de 100 $cm^3$. Après concentration à sec des fractions 1 à 48 à 40°C sous pression réduite (4 kPa), on obtient 21,2 g d'amino-2 (dimethyl-2,6 phénoxy)-7 naphtyridine-1,8 fondant à 218°C.

*Exemple 42*

On opère d'une manière analogue à celle décrite à l'exemple 1, mais à partir de 10,2 g d'acide méthoxy-4 benzoïque, de 10,9 g de N,N'-carbonyldiimidazole et de 13,4 g d'amino-2 (méthoxy-2 phénoxy)-7 naphthyridine-1,8. Le produit obtenu par précipitation dans l'eau (18 g; F = 167°C) est dissous dans 400 $cm^3$ d'éthanol bouillant. Après 2 heures de refroidissement à 4°C, le solide cristallisé est séparé par filtration, lavé par 2 fois 10 $cm^3$ d'éthanol et séché à 45°C sous pression réduite (0,067 kPa). On obtient 14 g de N-[(méthoxy-2 phénoxy)-7 naphtyridine-1,8 yl-2] méthoxy-4 benzamide fondant à 168-170°C.

*Exemple 43*

On opère d'une manière analogue à celle décrite à l'exemple 1, mais à partir de 15,9 g d'acide méthoxy-4 benzoïque, de 17 g de N,N'-carbonyldiimidazole et de 17,7 g d'amino-2 (méthoxy-3 phénoxy)-7 naphtyridine-1,8. Le produit obtenu par précipitation dans l'eau (12,1 g, F = 148°C) est purifié par filtration sur une colonne de 45 mm de diamètre contenant 180 g de silice (0,040 - 0,063 mm) en éluant par le dichlorométhane pur et en recueillant des fractions de 100 $cm^3$. Après concentration à sec des fractions 1 à 50 à 40°C sous pression réduite (4 kPa), on obtient 10 g d'un solide fondant à 148°C. Ce produit est dissous dans 130 $cm^3$ d'éthanol bouillant. Après 18 heures de refroidissement à 4°C, le solide cristallisé est séparé par filtration, lavé par 10 $cm^3$ d'éthanol et séché à 40°C sous pression réduite (0,067 kPa). On obtient 9 g de N-[(méthoxy-3 phénoxy)-7 naphtyridine-1,8 yl-2] méthoxy-4 benzamide fondant à 150°C.

L'amino-2 (méthoxy-3 phénoxy)-7 naphtyridine-1,8 peut être préparé de la façon suivante:

On opère d'une manière analogue à celle décrite à l'exemple 4, mais à partir de 17,95 g d'amino-2 chloro-7 naphtyridine-1,8, de 49,6 g de méthoxy-3 phénol et de 13,2 g de potasse en pastilles à 85%. Après traitement par la soude et lavage, le produit obtenu (23,8 g; F = 156°C) est dissous dans 200 $cm^3$ d'éthanol bouillant. Après 18 heures de refroidissement à 4°C, le solide cristallisé est séparé par filtration, lavé par 10 $cm^3$ d'éthanol et séché à 40°C sous pression réduite (0,067 kPa). On obtient 17,7 g d'amino-2 (méthoxy-3 phénoxy)- naphtyridine-1,8 fondant à 160°C.

*Exemple 44*

On opère d'une manière analogue à celle décrite à l'exemple 1, mais à partir de 9,5 g d'acide méthoxy-4 benzoïque, de 10,2 g de N,N'-carbonyldiimidazole et de 10,7 g d'amino-2 (méthoxy-4 phénoxy)-7 naphtyridine-1,8. Le produit obtenu par précipitation dans l'eau (14,3 g; F = 106°C) est purifié par chromatographie sur une colonne de 35 mm de diamètre contenant 200 g de silice (0,040 - 0,063 mm) en éluant par un mélange (98-2 en volumes) de chlorure de méthylène et de méthanol et en recueillant des fractions de 50 $cm^3$. Les fractions 22 à 28 sont concentrées à sec sous pression réduite (4 kPa) pour donner 10,8 g d'un solide fondant à 150°C. Ce produit est dissous dans 250 $cm^3$ d'éthanol bouillant. Après 3 heures de refroidissement à 4°C, le solide cristallisé est séparé par filtration, lavé par 2 fois 10 $cm^3$ d'éthanol et séché à 40°C sous pression réduite (0,067 kPa). On obtient 9 g de N-[(méthoxy-4 phénoxy)-7 naphtyridine-1,8 yl-2] méthoxy-4 benzamide fondant à 163°C.

*Exemple 45*

On opère d'une manière analogue à celle décrite à l'exemple 1, mais à partir de 3,6 g d'acide méthoxy-4 benzoïque, de 4,05 g de N,N'-carbonyldiimidazole et de 6,5 g d'amino-2 (pyridyl-3) oxy-7 naphtyridine-1,8. Le produit obtenu par précipitation dans l'eau (3,9 g; F environ 110°C) est purifié par chromatographie sur une colonne de 25 mm de diamètre contenant 80 g de silice (0,040 - 0,063 mm) en éluant par du chlorure de méthylène et en recueillant des fractions de 50 $cm^3$. Les fractions 16 à 28 sont réunies et concentrées à sec sous pression réduite (4 kPa) pour conduire à 3,2 g de solide cristallisé fondant à 174°C. Le produit isolé est dissous dans 125 $cm^3$ d'acétate d'éthyle bouillant. Après 2 heures de refroidissement à 4°C le solide cristallisé est séparé par filtration, lavé par 2 fois 3 $cm^3$ d'acétate d'éthyle et séché à 50°C sous pression réduite (0,067 kPa). On obtient 2,5 g de N-[(pyridyl-3) oxy-7 naphtyridine-1,8 yl-2] méthoxy-4 benzamide fondant à 184°C.

L'amino-2 (pyridyl-3) oxy-7 naphtyridine-1,8 peut être préparée de la façon suivante:

On opère d'une manière analogue à celle décrite à l'exemple 4, mais à partir de 17,9 g d'amino-2 chloro-7 naphtyridine-1,8, de 38 g d'hydroxy-3 pyridine et de 11,2 g de potasse en pastilles à 85%. Le produit obtenu par précipitation dans l'eau et extraction par l'acétate d'éthyle (4,7 g) est dissous dans 80 $cm^3$ d'acétate d'éthyle bouillant. Après 3 heures de refroidissement à 4°C, le solide cristallisé est séparé par filtration, lavé par 2 fois 5 $cm^3$ d'éther éthylique et séché à l'air à 20°C. On obtient 2,1 g d'amino-2 (pyridyl-3) oxy-7 naphtyridine-1,8 fondant à 178°C.

*Exemple 46*

On opère d'une manière analogue à celle décrite à l'exemple 1, mais à partir de 4,6 g d'acide méthoxy-4 benzoïque, de 4,9 g de N,N'-carbonyldiimidazole et de 5,1 g d'amino-2 (méthyl-1 pipéridyl-4) oxy-7 naphtyridine-1,8. Le produit obtenu par précipitation dans l'eau (6,5 g; F environ 80°C) est dissous dans 100 $cm^3$ de chlorure de méthylène, le produit est extrait par 100 $cm^3$ d'acide chlorhydrique 2N, la phase aqueuse obtenue est lavée par 2 fois 50 $cm^3$ de chlorure de méthylène et neutralisée par 50 $cm^3$ de soude 4N en présence de 100 $cm^3$ de chlorure de méthylène. Après lavage à l'eau et séchage, la phase organique est concentrée à sec à 40°C sous pression réduite (4 kPa) pour donner 6,3 g d'un produit amorphe. Ce produit est dissous dans 50 $cm^3$ de méthylcyclohexane bouillant. Après 1 heure de refroidissement à 20°C, le solide cristallisé est séparé par filtration, lavé par 20 $cm^3$ de méthylcyclohexane et séché à 50°C sous pression réduite (0,067 kPa). On obtient 5,6 g de N-[(méthyl-2 pipéridyl-4) oxy-7 naphthyridine-1,8 yl-2] méthoxy-4 benzamide fondant à 200°C.

L'amino-2 (méthyl-1 pipéridyl-4) oxy-7 naphtyridine-1,8 peut être préparée de la façon suivante:

On opère d'une manière analogue à celle décrite à l'exemple 29, mais à partir de 27 g d'amino-2 chloro-7 naphtyridine-1,8 de 71 g d'hydroxy-4 méthyl-1 pipéridine et de 8 g de sodium. Après traitement à l'eau, le mélange réactionnel est extrait par du chlorure de méthylène pour donner 33,4 g d'un solide amorphe. Ce solide est purifié par chromatographie sur une colonne de 60 mm de diamètre contenant 335 g de silicie (0,040 - 0,063 mm) en éluant par un mélange (90-10 en volumes) de chlorure de méthylène et de méthanol et en recueillant des fractions de 100 $cm^3$. Les fractions 12 à 35 sont concentrés à sec sous pression réduite (4 kPa) pour donner 10,4 g d'un solide fondant environ à 100°C. Ce produit est dissous dans 50 $cm^3$ de cyclohexane bouillant. Après 1 heure de refroidissement à 4°C, le solide cristallisé est séparé par filtration, lavé par 20 $cm^3$ de cyclohexane et séché à 40°C sous pression réduite (0,067 kPa). On obtient 4,3 g d'amino-2 (méthyl-1 pipéridyl-4) oxy-7 naphtyridine-1,8 fondant à 174°C.

*Exemple 47*

On opère d'une manière analogue à celle décrite à l'exemple 1, mais à partir de 13,3 g d'acide éthoxy-4 benzoïque, de 12,9 g de N,N'-carbonyldiimidazole et de 8,9 g d'amino-2 chloro-7 naphtyridine-1,8. Le produit obtenu par précipitation dans l'eau (15,3 g; F environ 170°C) est dissous dans 500 $cm^3$ d'acétonitrile bouillant. Après 16 heures de refroidissement à 4°C le solide cristallisé est séparé par filtration, lavé par 3 fois 25 $cm^3$ d'acétonitrile et séché à 40°C sous pression réduite (0,067 kPa).

On obtient 8 g de N-(chloro-7 naphtyridine-1,8 yl-2) éthoxy-4 benzamide fondant à 208°C.

*Exemple 48*

On opère d'une manière analogue à celle décrite à l'exemple 1, mais à partir de 8 g d'acide éthoxy-4 benzoïque, de 7,8 g de N,N'-carbonyldiimidazole et de 5,6 g d'amino-2 méthoxy-7 naphtyridine-1,8. Le produit obtenu par précipitation dans l'eau (9,4 g; F = 74°C) est dissous dans 94 $cm^3$ d'acétonitrile bouillant. Après 3 heures de refroidissement à 4°C le solide cristallisé est séparé par filtration, lavé par 3 fois 20 $cm^3$ d'acétonitrile et séché à 30°C sous pression réduite (0,067 kPa). On obtient 7 g de N-(méthoxy-7 naphtyridine-1,8 yl-2) éthoxy-4 benzamide fondant à 145°C.

*Exemple 49*

On opère d'une manière analogue à celle décrite à l'exemple 1, mais à partir de 15,5 g d'acide butoxy-4 benzoïque, de 12,9 g de N,N'-carbonyldiimidazole et de 8,9 g d'amino-2 chloro-7 naphtyridine-1,8. Le produit obtenu par précipitation dans l'eau (environ 18 g; F = 140°C) est purifié par chromatographie sur une colonne de 40 mm de diamètre chargée avec 200 g de silice (0,063 - 0,2 mm) en utilisant comme éluant le chlorure de méthylène et en recueillant des fractions de 60 $cm^3$. Après concentration à sec des fractions 8 à 12 à 40°C sous pression réduite (4 kPa) on obtient 6,2 g de N-(chloro-7 naphthyridine-1,8 yl-2) butoxy-4 benzamide fondant à 197°C.

*Exemple 50*

On opère d'une manière analogue à celle décrite à l'exemple 1, mais à partir de 16,6 g d'acide méthylènedioxy-3,4 benzoïque, de 16,2 g de N,N'-carbonyldiimidazole et de 11,7 g d'amino-2 chloro-7 naphtyridine-1,8. Le produit obtenu par précipitation dans l'eau (15,7 g; F = 256°C) est dissous dans 1000 $cm^3$ de propanol-1. Après 16 heures de refroidissement à 4°C le solide cristallisé est séparé par filtration, lavé par 3 fois 20 $cm^3$ de propanol-1 et séché à 40°C sous pression réduite (0,067 kPa). On obtient 8,4 g de N-(chloro-7 naphtyridine-1,8 yl-2) méthylènedioxy-3,4 benzamide fondant à 256°C.

*Exemple 51*

On opère d'une manière analogue à celle décrite à l'exemple 1, mais à partir de 6,6 g d'acide diméthylamino-3 benzoïque, de 6,5 g de N,N'-carbonyldiimidazole et de 5,4 g d'amino-2 chloro-7 naphtyridine-1,8. Le produit obtenu par précipitation dans l'eau (9 g; F = 212°C) est dissous dans 400 $cm^3$ d'acétonitrile bouillant. Après 3 heures de refroidissement à 4°C, le solide cristallisé est séparé par filtration, lavé par 3 fois 20 $cm^3$ d'acétonitrile et séché à 40°C sous pression réduite (0,067 kPa). On obtient 6,2 g de N-(chloro-7 naphtyridine-1,8 yl-2) diméthylamino-3 benzamide fondant à 215°C.

*Exemple 52*

On opère d'une manière analogue à celle décrite à l'exemple 1, mais à partir de 18,6 g d'acide diméthylamino-3 benzoïque, de 17,8 g de N,N'-carbonyldiimidazole et de 12,3 g d'amino-2 méthoxy-7 naphtyridine-1,8. Le produit obtenu par précipitation dans l'eau (24,7 g; F = 82-84°C) est purifié par chromatographie sur une colonne de 4,5 cm de diamètre contenant 220 g de silice (0,040 - 0,063 mm) en éluant par un mélange (98-2 en volume) de dichlorométhane et de méthanol. On recueille des fractions de 100 $cm^3$, les fractions 7 à 22 sont réunies et concentrées à 40°C sous pression réduite (4 kPa) pour donner 24 g d'un solide fondant à 90°C. Ce produit est dissous dans 120 $cm^3$ d'acétonitrile bouillant. Après 1 heure de refroidissement à 4°C, le solide cristallisé est séparé par filtration, lavé par 2 fois 10 $cm^3$ d'oxyde d'isopropyle et séché à 40°C sous pression réduite (0,5 mm de mercure). On obtient 5,7 g de N-(méthoxy-7 naphtyridine -1,8 yl-2) diméthylamino-3 benzamide fondant à 130°C.

*Exemple 53*

En opérant d'une manière analogue à celle décrite à l'exemple 1, mais à partir de 23,1 g d'acide diméthylamino-3 benzoïque, de 22,7 g de N,N'-carbonyldiimidazole et de 23,7 g d'amino-2 phénoxy-7 naphtyridine-1,8. Le produit obtenu par précipitation dans l'eau (44 g; F environ 90°C) est dissous dans 1000 $cm^3$ d'acétonitrile bouillant. Après 3 heures de refroidissement à 4°C, le solide cristallisé est séparé par filtration, lavé par 3 fois 50 $cm^3$ d'acétonitrile et séché à 40°C sous pression réduite (0,067 kPa). On obtient 32 g de N-(phénoxy-7 naphtyridine-1,8 yl-2) diméthylamino-3 benzamide fondant à 120°C.

*Exemple 54*

On opère d'une manière analogue à celle décrite à l'exemple 1, mais à partir de 3,7 g d'acide nicotinique, de 4,9 g de N,N'-carbonyldiimidazole et de 3,6 g d'amino-2 chloro-7 naphtyridine-1,8. Le produit précipité dans le mélange est séparé par filtration, lavé par 3 fois 20 $cm^3$ de tétrahydrofuranne, 3 fois 50 $cm^3$ d'eau et est ensuite séché à 50°C sous pression réduite (0,067 kPa). Le produit obtenu (4,75 g; F = 200°C) est dissous dans 435 $cm^3$ d'acétonitrile bouillant. Après 2 heures de refroidissement à 4°C

le solide cristallisé est séparé par filtration, lavé par 3 fois 20 $cm^3$ d'acétonitrile et séché à 50°C sous pression réduite (0,067 kPa). On obtient 3,9 g de N-(chloro-7 naphtyridine-1,8 yl-2) pyridylcarboxamide-3 fondant à 200°C.

*Exemple 55*

On opère d'une manière analogue à celle décrite à l'exemple 1, mais à partir de 7,4 g d'acide nicotinique de 9,7 g de N,N'-carbonyldiimidazole et de 7 g d'amino-2 méthoxy-7 naphtyridine-1,8. Après filtration, lavage à l'eau et séchage, le solide cristallisé formé au cours de la réaction (10,3 g; F = 245°C) est dissous dans 900 $cm^3$ de propanol-1 bouillant. Le solide cristallisé après 3 heures de refroidissement à 4°C est séparé par filtration, lavé par 3 fois 20 $cm^2$ de propanol-1 et séché à 50°C sous pression réduite (0,067 kPa). On obtient 8,7 g de N-(méthoxy-7 naphtyridine-1,8 yl-2) pyridylcarboxamide-3 fondant à 247°C.

*Exemple 56*

On opère d'une manière identique à celle décrite à l'exemple 1, mais à partir de 1,7 g d'acide méthoxy-6 pyridine carboxylique-3, de 1,8 g de N,N'-carbonyldiimidazole et de 1,95 g d'amino-2 chloro-7 naphtyridine-1,8. Le produit obtenu (2,5 g; F = 270°C) est dissous dans 190 $cm^3$ d'un mélange diméthylformamide méthanol (1/6).

Après 1 heure de refroidissement à 4°C, le solide cristallisé est séparé par filtration, lavé par 2 fois 10 $cm^3$ de méthanol et séché à 40°C sous pression réduite (0,066 kPa). On obtient 2,1 g de N-(chloro-7 naphtyridine-1,8 yl-2) méthoxy-6 pyridinecarboxamide-3 fondant à 270°C.

L'acide méthoxy-6 pyridinecarboxylique-3 peut être préparé de la manière suivante:

A une suspension de 4,15 g d'acide chloro-6 pyridinecarboxylique-3 dans 40 $cm^3$ de méthanol, on ajoute 40 $cm^3$ d'une solution méthanolique 4M de méthylate de sodium. Le mélange est chauffé à reflux pendant 60 heures. Le solvant est distillé sous pression réduite (4 kPa). Le résidu est repris par 100 $cm^3$ d'eau distillée, et le mélange est acidifié à pH 5 par de l'acide chlorhydrique en solution aqueuse 11,9M. Le précipité obtenu est séparé par filtration, lavé par 5 fois 10 $cm^3$ d'eau distillée et séché à l'air. On obtient 3,3 g d'acide méthoxy-6 pyridinecarboxylique-3 fondant à 180°C.

*Exemple 57*

On opère d'une manière analogue à celle décrite à l'exemple 1, mais à partir de 4,7 g d'acide méthoxy-6 pyridinecarboxylique-3, de 4,9 g de N,N'-carbonyldiimidazole et de 9,1 g d'amino-2 phénoxy-7 naphthyridine-1,8. Le produit obtenu par précipitation dans l'eau (11 g; F = 115°C) est dissous dans 50 $cm^3$ d'acétonitrile bouillant. Après 10 heures de refroidissement à 20°C le solide cristallisé est séparé par filtration, lavé par 3 fois 10 $cm^3$ de diisopropyléther et séché à 30°C sous pression réduite (0,067 kPa). On obtient 7 g de N-(phénoxy-7 naphtyridine-1,8 yl-2 méthoxy-6 pyridinecarboxamide-3 fondant à 115-120°C.

*Exemple 58*

On opère d'une manière analogue à celle décrite à l'exemple 1, mais à partir de 9 g d'acide thiophènecarboxylique-2, de 11,3 g de N,N'-carbonyldiimidazole et de 9,5 d d'amino-2 chloro-7 naphtyridine-1,8. Le produit obtenu par précipitation dans l'eau (9,6 g; F = 242°C) est dissous dans 700 $cm^3$ d'éthanol bouillant. Après 1 heure de refroidissement à 4°C le solide cristallisé est séparé par filtration, lavé par 2 fois 25 $cm^3$ d'éthanol et séché à 40°C sous pression réduite (0,067 kPa). On obtient 7,5 g de N-(chloro-7 naphtyridine-1,8 yl-2) thiophènecarboxamide-2 fondant à 244°C.

*Exemple 59*

On opère d'une manière analogue à celle décrite à l'exemple 1, mais à partir de 16 g d'acide thiophènecarboxylique-3, de 20,1 g de N,N'-carbonyldiimidazole et de 16,8 g d'amino-2 chloro-7 naphtyridine-1,8. Le produit obtenu par précipitation dans l'eau (10,6 g; F = 250°C) est dissous dans 980 $cm^3$ d'acétonitrile bouillant. Après 2 heures de refroidissement à 4°C, le solide cristallisé est séparé par filtration, lavé par 2 fois 25 $cm^3$ d'acétonitrile et séché à 40°C sous pression réduite (0,067 kPa). On obtient 7,5 g de N-(chloro-7 naphtyridine-1,8 yl-2) thiophènecarboxamide-3 fondant à 254°C.

*Exemple 60*

On opère d'une manière analogue à celle décrite à l'exemple 1, mais à partir de 14 g d'acide thiophène-2 carboxylique, de 17,8 g de N,N'-carbonyldiimidazole et de 12,3 g d'amino-2 méthoxy-7 naphtyridine-1,8. Le produit obtenu par précipitation dans l'eau (19,5 g; F = 216°C) est dissous dans 750 $cm^3$ d'éthanol bouillant. Après 3 heures de refroidissement à 4°C, le solide cristallisé est séparé par filtration, lavé par 2 fois 25 $cm^3$ d'éthanol et séché à 40°C sous pression réduite (0,067 kPa). On obtient 15,6 g de N-(méthoxy-7 naphtyridine-1,8 yl-2) thiophènecarboxamide-2 fondant à 220°C.

*Exemple 61*

On opère d'une manière analogue à celle décrite à l'exemple 1, mais à partir de 4 g d'acide thiophènecarboxylique-2, de 5,1 g de N,N'-carbonyldiimidazole et de 7 g d'amino-2 chloro-7 naphtyridine-1,8. Le produit obtenu par précipitation dans l'eau (8,8 g; F = 175°C) est dissous dans 150 $cm^3$ de méthanol bouillant. Après 3 heures de refroidissement à 4°C le solide cristallisé est séparé par filtration, lavé par 3 fois 10 $cm^3$ de méthanol et séché à 40°C sous pression réduite (0,067 kPa). On obtient 6 g de N-(phénoxy-7 naphtyridine-1,8 yl-2) thiophènecarboxamide-2 fondant à 176°C.

*Exemple 62*

On opère d'une manière analogue à celle décrite à l'exemple 1, mais à partir de 5 g d'acide thiophènecarboxylique-3, de 6,3 g de N,N'-carbonyldiimidazole et de 8,3 g d'amino-2 phénoxy-7 naphtyridine-1,8. Le produit obtenu par précipitation dans l'eau (11,6 g; F = 110°C) est dissous dans 50 $cm^3$ d'acétonitrile bouillant. Après 2 heures de refroidissement à 4°C, le solide cristallisé est séparé par

filtration, lavé par 3 fois 10 $cm^3$ de diisopropyle éther et séché à 35°C sous pression réduite (0,067 kPa). On obtient 8,2 g de N-(phénoxy-7 naphtyridine-1,8 yl-2) thiophènecarboxamide-3 fondant à 95°C.

*Exemple 63*

On opère de manière identique à celle décrite à l'exemple 1 mais à partir de 4,1 g d'acide méthyl-5 thiophènecarboxylique-2, de 5,6 g de N,N'-carbonyldiimidazole et de 4,85 g d'amino-2 chloro-7 naphtyridine-1,8. Le produit obtenu (3,8 g; F = 220°C) est dissous dans 150 $cm^3$ d'acétonitrile bouillant. Après 4 heures de refroidissement à 4°C le solide cristallisé est séparé par filtration, lavé par 2 fois 10 $cm^3$ d'acétonitrile et séché à 40°C sous pression réduite (0,066 kPa). On obtient 3,1 g de N-(chloro-7 naphtyridine-1,8 yl-2) méthyl-5 thiophènecarboxamide-2 fondant à 222°C.

*Exemple 64*

On opère d'une manière analogue à celle décrite à l'exemple 1, mais à partir de 8,95 g d'acide furannecarboxylique-2, de 12,8 g de N,N'-carbonyldiimidazole et de 10,5 g d'amino-2 méthoxy-7 naphtyridine-1,8. Le produit obtenu par précipitation dans l'eau (15,5 g; F = 199°C) est dissous dans 500 $cm^3$ d'éthanol bouillant. Après 2 heures de refroidissement à 4°C le solide cristallisé est séparé par filtration, lavé par 2 fois 10 $cm^3$ d'éthanol et séché à 45°C sous pression réduite (0,067 kPa). On obtient 13,5 g de N-(méthoxy-7 naphthyridine-1,8 yl-2) furannecarboxamide-2 fondant à 201°C.

*Exemple 65*

On opère d'une manière analogue à celle décrite à l'exemple 1, mais à partir de 7,5 g d'acide furannecarboxylique-2, de 10,9 g de N,N'-carbonyldiimidazole et de 11,9 g d'amino-2 phénoxy-7 naphtyridine-1,8. Le produit obtenu par précipitation dans l'eau (15,7 g; F = 164°C) est dissous dans 200 $cm^3$ d'éthanol bouillant. Après 2 heures de refroidissement à 4°C, le solide cristallisé est séparé par filtration, lavé par 15 $cm^3$ d'éthanol et séché à 40°C sous pression réduite (0,067 kPa). On obtient 14,4 g de N-(phénoxy-7 naphtyridine-1,8 yl-2) furannecarboxamide-2 fondant à 167°C.

*Exemple 66*

On opère d'une manière analogue à celle décrite à l'exemple 1, mais à partir de 7,5 g d'acide furanne-3 carboxylique, de 10,9 g de N,N'-carbonyldiimidazole et de 11,9 g d'amino-2 phénoxy-7 naphtyridine-1,8. Le produit obtenu par précipitation dans l'eau (13 g; F environ 90°C) est dissous dans 80 $cm^3$ d'acétonitrile bouillant. Après 2 heures de refroidissement à 4°C le solide cristallisé est séparé par filtration, lavé par 2 fois 10 $cm^3$ d'acétonitrile et séché à 40°C sous pression réduite (0,067 kPa). On obtient 10,7 g de N-(phénoxy-7 naphtyridine-1,8 yl-2) furannecarboxamide-3 fondant à 102°C.

*Exemple 67*

A une solution de 12 g d'acide méthyl-6 pyridazine carboxylique-3 dans 250 $cm^3$ de tétrahydrofuranne anhydre, on ajoute 20 g de N,N'-carbonyldiimidazole. On observe un lent dégagement gazeux. Le mélange est agité à une température voisine de 20°C jusqu'à fin de dégagement gazeux. On ajoute ensuite 11 g d'amino-2 chloro-7 naphtyridine-1,8 et on chauffe à reflux pendant 20 heures. Le précipité obtenu est séparé par filtration, lavé à l'éthanol, séché à l'air puis extrait par 1000 $cm^3$ d'isopropanol au reflux dans un extracteur solide-liquide pendant 20 heures. Après 2 heures de refroidissement à 4°C de la solution isopropanolique ainsi obtenue, le solide cristallisé est séparé par filtration puis dissous dans 300 $cm^3$ de diméthylformamide chaud (120°C).

Après 3 heures de refroidissement à 4°C, le solide cristallisé est séparé par filtration, lavé par 2 fois 20 $cm^3$ de méthanol et séché à 40°C sous pression réduite (0,066 kPa). On obtient 4,5 g de N-(chloro-7 naphtyridine-1,8 yl-2) méthyl-6 pyridazinecarboxamide-3 fondant à 258°C.

*Exemple 68*

On opère d'une manière analogue à celle décrite à l'exemple 8, mais à partir de 10,7 g d'amino-2 chloro-7 quinoléine dans 120 $cm^3$ de pyridine anhydre et de 15,5 g de chlorure de benzoyle. Le produit obtenu par précipitation dans l'eau (16 g; F = 127°C) est isolé par filtration puis dissous dans 1000 $cm^3$ de cyclohexane bouillant. Après 1 heure de refroidissement à 4°C, le solide cristallisé est séparé par filtration et séché à 45°C sous pression réduite (0,067 kPa). On obtient 12,9 g d'un produit fondant à 130°C qui est à nouveau cristallisé dans 60 $cm^3$ d'acétone bouillante. Après 1 heure de refroidissement à 4°C, le solide recristallisé est séparé par filtration, lavé par 2 fois 5 $cm^3$ d'acétone et séché à 40°C sous pression réduite (0,067 kPa). On obtient 9 g de N-(chloro-7 quinolyl-2) benzamide fondant à 133°C.

*Exemple 69*

A une solution de 6,65 g de N-(formyl-7 naphtyridine-1,8 yl-2) méthoxy-4 benzamide dans 150 $cm^3$ de méthanol, on ajoute en 15 minutes, à 20°C, 0,41 g de borohydrure de sodium puis on laisse agiter pendant 1 heure à 20°C jusqu'à la fin du dégagement gazeux. La suspension obtenue est versée sur 800 $cm^3$ d'eau distillé qui est acidifiée à pH = 4 à l'aide d'acide acétique. Le solide obtenu par filtration et séchage (6,15 g; F = 212°C) est dissous dans 180 $cm^3$ de dioxanne bouillant. Après refroidissement pendant 3 heures à une température voisine de 20°C, le solide cristallisé est séparé par filtration, lavé par 2 fois 15 $cm^3$ de dioxanne et séché à 50°C sous pression réduite (0,067 kPa). On obtient 4,4 g de N-(hydroxyméthyl-7 naphtyridine-1,8 yl-2) méthoxy-4 benzamide fondant à 218°C.

Le N-(formyl-7 naphtyridine-1,8 yl-2) méthoxy-4 benzamide peut être préparé de la façon suivante:

A une solution de 29,3 g de N-(méthyl-7 naphtyridine-1,8 yl-2) méthoxy-3 benzamide dans 1000 $cm^3$ de dioxanne, on ajoute 33,1 g d'oxyde de sélénium puis on chauffe pendant 3 heures à reflux. La suspension obtenue est filtrée à chaud, le filtrat est repris par 2500 $cm^3$ de chlorure de méthylène et lavé par 5 fois 1000 $cm^3$ d'eau distillée. La solution

organique est séchée et concentrée à sec sous pression réduite (4 kPa) pour donner 11,1 g d'un produit fondant à 190°C qui est dissous dans 500 $cm^3$ d'acétonitrile bouillant. Après refroidissement pendant 2 heures à 4°C, le solide cristallisé est séparé par filtration, lavé par 2 fois 25 $cm^3$ d'acétonitrile et séché à 50°C sous pression réduite (0,067 kPa). On obtient 6,65 g de N-(formyl-7 naphtyridine-1,8 yl-2) méthoxy-4 benzamide fondant à 194°C.

*Exemple 70*

A une solution de 15 g de chloro-2 phénoxycarbonylamino-7 naphtyridine-1,8 dans 500 $cm^3$ de tétrahydrofuranne anhydre, on ajoute 500 $cm^3$ d'acétonitrile anhydre et 4,2 g de tétrahydro-1,2,5,6 pyridine. On chauffe à reflux pendant 45 minutes. La solution est concentrée sous pression réduite (4 kPa) jusqu'à un volume de 100 $cm^3$. Après 2 heures de refroidissement à 4°C le solide cristallisé est séparé par filtration, lavé par 3 fois 15 $cm^3$ d'acétonitrile et séché à 40°C sous pression réduite (0,067 kPa). On obtient 10,5 g de N-(chloro-7 naphtyridine-1,8 yl-2) tétrahydro-1,2,5,6 pyridinecarboxamide-1 fondant à 173°C.

La chloro-2 phénoxycarbonylamino-7 naphtyridine-1,8 peut être préparée de la manière suivante:

A une solution de 9,2 g d'amino-2 chloro-7 naphtyridine-1,8 dans 130 $cm^3$ de pyridine, maintenue à une température voisine de 20°C, on ajoute en 15 minutes 9,6 g de chloroformiate de phényle. Le mélange est agité pendant 3 heures à une température voisine de 20°C. Le mélange est versé dans 1000 $cm^3$ d'eau distillée. Le précipité formé est séparé par filtration et lavé à l'eau.

Le produit obtenu (25 g; F environ 200°C) est dissous dans 500 $cm^3$ d'acétonitrile bouillant. Après 3 heures de refroidissement à 4°C, le solide cristallisé est séparé par filtration, lavé par 3 fois 50 $cm^3$ d'acétonitrile et séché à 40°C sous pression réduite (0,067 kPa). On obtient 7,4 g de chloro-2 phénoxycarbonylamino-7 naphtyridine-1,8 fondant à 208°C.

La présente invention concerne également les compositions pharmaceutiques qui contiennent les produits de formule (I) à l'état pur ou en association avec un adjuvant, un diluant et/ou un enrobage compatibles et pharmaceutiquement acceptables. Ces médicaments peuvent être employés par voie orale, rectale, parentérale ou percutanée.

Comme compositions solides pour administration orale peuvent être utilisés des comprimés, des pilules, des poudres (généralement dans des capsules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrificant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les composants selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule fovorisant la migration percutanée.

Les médicaments et compositions selon l'invention sont particulièrement utiles en thérapeutique humaine pour leur action anxiolytique, hypnotique, anticonvulsivante, antiépileptique et myorelaxante et, le cas échéant, pour leur action anti-infectieuse et immunorestauratrice.

En thérapeutique humaine, les doses dépendent de l'effet recherché et de la durée du traitement; elles sont généralement comprises entre 10 et 500 mg par jour par voie orale pour un adulte.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent une composition selon l'invention.

*Exemple A*

On prépare selon la technique habituelle des comprimés dosés à 10 mg de produit actif ayant la composition suivante:

| | |
|---|---|
| — N-chloro-7 naphtyridine-1,8 yl-2) méthoxy-4 benzamide | 0,010 g |
| — amidon | 0,200 g |
| — silice précipitée | 0,036 g |
| — stéarate de magnesium | 0,004 g |

*Exemple B*

On prépare selon la tecnique habituelle des comprimés dosés à 10 mg de produit actif ayant la composition suivante:

| | |
|---|---|
| — N-(chloro-7 naphtyridine-1,8 yl-2) fluoro-4 benzamide | 0,010 g |
| — amidon | 0,200 g |
| — silice précipitée | 0,036 g |
| — stéarate de magnésium | 0,004 g |

*Exemple C*

On prépare selon la technique habituelle des comprimés dosés à 10 mg de produit actif ayant la composition suivante:

| | |
|---|---|
| — N-(méthoxy-7 naphtyridine-1,8 yl-2) cyclopropanecarboxamide | 0,010 g |
| — amidon | 0,200 g |
| — silice précipitée | 0,036 g |
| — stéarate de magnésium | 0,004 g |

*Exemple D*

On prépare selon la technique habituelle des comprimés dosés à 10 mg de produit actif ayant la composition suivante:

| | |
|---|---|
| — N-(méthoxy-7 naphtyridine-1,8 yl-2) thiophènencarboxamide-2 | 0,010 g |
| — amidon | 0,200 g |
| — silice précipitée | 0,036 g |
| — stéarate de magnésium | 0,004 g |

**Revendications pour les Etats contractants:**
BE, CH, DE, FR, GB, IT, LI, LU, NL, SE

1. Un dérivé d'amide substitué caractérisé en ce qu'il répond à la formule générale:

R-CONH-Het

dans laquelle le symbole R représente un radical cycloalcoyle contenant 3 à 6 atomes de carbone, cyclohexadiényle, phényle, phényle substitué par 1 ou 2 atomes de fluor ou par un radical hydroxy, ou substitué en position -3 ou -4 par un radical alcoyle ou alcoyloxy, ou en positions -3 et -4 par un radical méthylènedioxy, ou substitué en position -2 ou -3 par un radical dialcoylamino, ou bien le symbole R représente un radical hétérocyclyle choisi parmi pyridyle-3, alcoyloxypyridyle-3, thiényle, alcoylthiényle, furyle, tétrahydropyridyle, pyridazinyle et alcoylpyridazinyle, et le symbole Het représente un radical quinolyle-2 ou naphtyridine-1,8 yle-2 éventuellement substitués en position -7 par un atome d'halogène, par un radical hydroxyméthyle, par un radical alcoyle, alcoyloxy, alcoyloxyalcoyloxy, par un radical alcényloxy ou alcynyloxy contenant 3 ou 4 atomes de carbone, par un radical alcoylthio ou benzylthio, par un radical phénoxy (éventuellement substitué par un atome de fluor ou substitué par un atome de chlore ou de brome en position -2, ou substitué par un radical alcoyloxy ou par 1 ou 2 radicaux alcoyle) ou par un radical pyridyloxy ou alcoylpipéridyloxy, étant entendu que, lorsque Het représente un radival quinolyle-2, R est autre que phényle et que les radicaux ou portions alcoyles cités ci-dessus sont droits ou ramifiés et contiennent 1 à 4 atomes de carbone.

2. Un dérivé d'amide selon la revendication 1, caractérisé en ce que le symbole R est un radical cyclopropyle, cyclobutyle, cyclohexadiène-1,4 yle-1, phényle, phényle substitué par 1 ou 2 atomes de fluor ou par un radical hydroxy, ou substitué en position -3 ou -4 par un radical alcoyle contenant 1 ou 2 atomes de carbone ou alcoyloxy contenant 1 à 4 atomes de carbone, ou en positions -3 et -4 par un radical méthylènedioxy ou substitué par un radical diméthylamino en position -3, ou bien le symbole R est un radical hétérocyclyle choisi parmi pyridyle-3, alcoyloxy-6 pyridyle-3, thiényle, alcoyl-5 thiényle, furyle pyridazinyle-3 ou alcoyl-6 pyridazinyle-3 dont les portions alcoyle contiennent 1 ou 2 atomes de carbone et le symbole Het représente un radical quinolyle-2 ou naphtyridine-1,8 yle-2 éventuellement substitués en position -7 par un atome d'halogène, par un radical hydroxyméthyle, par un radical alcoyle ou alcoylthio contenant 1 ou 2 atomes de carbone, par un radical alcoyloxy contenant 1 à 4 atomes de carbone, ou alcoyloxyalcoyloxy contenant 2 à 4 atomes de carbone, par un radical allyloxy, propargyloxy ou benzylthio, par un radical phénoxy (éventuellement substitué par un atome de fluor ou par un atome de chlore ou de brome en position -2, par un radical alcoyloxy ou par 1 ou 2 radicaux alcoyle contenant 1 ou 2 atomes de carbone) ou par un radical pyridyl-3 oxy ou alcoyl-1 pipéridyl-4 oxy étant entendu que lorsque le symbole Het représente un radical quinolyle-2, R est autre que phényle.

3. Un dérivé d'amide selon la revendication 1, caractérisé en ce que le symbole R est un radical cyclopropyle, cyclohexadiène-1,4 yle-1, phényle, phényle substitué par 1 ou 2 atomes de fluor ou par un radical hydroxy, ou substitué en position -3 ou -4 par un radical alcoyloxy contenant 1 ou 2 atomes de carbone ou en positions -3 et -4 par un radical méthylènedioxy, ou substitué en position -3 par un radical diméthylamino, ou bien le symbole R est un radical hétérocyclyle choisi parmi pyridyle-3, alcoyloxy-6 pyridyle-3 dont la portion alcoyle contient 1 ou 2 atomes de carbone, thiényle ou furyle et le symbole Het représente un radical naphthyridine-1,8 yle-2 éventuellement substitué en position -7 par un atome d'halogène, par un radical alcoyle, alcoyloxy ou alcoylthio contenant 1 ou 2 atomes de carbone, par un radical alcoyloxyalcoyloxy contenant 2 à 4 atomes de carbone, par un radical allyloxy ou phénoxy (éventuellement substitué par un atome de fluor, ou par un atome de chlore ou de brome en position-2, par un radical alcoyloxy ou par 1 ou 2 radicaux alcoyle contenant 1 ou 2 atomes de carbone), ou par un radical pyridyl-3 oxy.

4. Un dérivé d'amide selon la revendication 1, caractérisé en ce que le symbole R est un radical cyclopropyle, phényle, phényle substitué en position -3 ou -4 par un atome de fluor, ou substitué par 2 atomes de fluor ou par un radical hydroxy, ou substitué en position -3 ou -4 par un radical méthoxy, ou bien le symbole R est un radical hétérocyclyle choisi parmi alcoyloxy-6 pyridyle-3 dont la portion alcoyle contient 1 ou 2 atomes de carbone, thiényle-2 ou furyle-3, et le symbole Het représente un radical naphtyridine-1,8 yle-2 substitué en position -7 par un atome d'halogène, par un radical méthoxy ou

par un radical phénoxy (éventuellement substitué par un atome de fluor ou en position −2 par un atome de chlore ou un radical méthyle, ou en position −2 ou −3 par un radical méthoxy), ou le symbole R représente un radical cyclohexadiène-1,4 yle-1, phényle (substitué en positions −3 et −4 par un radical méthylènedioxy ou substitué en position −3 par un radical diméthylamino) ou un radical pyridyle-3 et le symbole Het représente un radical naphtyridine-1,8 yle-2 substitué en position −7 par un atome d'halogène.

5. Un procédé de préparation d'un produit selon la revendication 1, caractérisé en ce que l'on fait agir un acide de formule générale:

R-COOH

dans laquelle R est défini comme dans la revendication 1, ou un dérivé réactif de cet acide sur une amine de formule générale:

$H_2N$-Het

dans laquelle Het est défini comme dans la revendication 1, puis le cas échéant élimine les radicaux protecteurs.

6. Un procédé selon la revendication 2 caractérisé en ce que l'on fait réagir un dérivé réactif de l'acide choisi parmi l'anhydride de l'acide, un anhydride mixte, un halogénure d'acide ou un ester.

7. Un procédé de préparation d'un produit selon la revendication 1, pour lequel le symbole Het contient un substituant hydroxyméthyle, caractérisé en ce que l'on réduit l'aldéhyde correspondant.

8. Un procédé de préparation d'un produit selon la revendication 1, pour lequel le symbole R est un radical tétrahydropyridyle, caractérisé en ce que l'on fait agir la tétrahydropyridine sur un carbamate de formule générale:

$C_6H_5OCONH$-Het

dans laquelle le symbole Het est défini comme dans la revendication 1.

9. Composition pharmaceutique caractérisé en ce qu'elle comprend au moins un produit selon la revendication 1, à l'état pur, ou en association avec tout autre diluant ou adjuvant compatible et pharmaceutiquement acceptable.

**Revendications pour l'Etat contractant:** AT

Procédé de préparation d'un nouveau dérivé d'amide substitué de formule générale:

R-CONH-Het

dans laquelle le symbole R représente un radical cycloalcoyle contenant 3 à 6 atomes de carbone, cyclohexadiényle, phényle, phényle substitué par 1 ou 2 atomes de fluor ou par un radical hydroxy, ou substitué en position −3 ou −4 par un radical alcoyle ou alcoyloxy, ou en positions −3 et −4 par un radical méthylènedioxy, ou substitué en position −2 ou −3 par un radical dialcoylamino, ou bien le symbole R représente un radical hétérocyclyle choisi parmi pyridyle-3, alcoyloxypyridyle-3, thiényle, alcoylthiényle, furyle, tétrahydropyridyle, pyridazinyle et alcoylpyridazinyle, et le symbole Het représente un radical quinolyle-2 ou naphtyridine-1,8 yle-2 éventuellement substitués en position −7 par un atome d'halogène, par un radical hydroxyméthyle, par un radical alcoyle, alcoyloxy, alcoyloxyalcoyloxy, par un radical alcényloxy ou alcynyloxy contenant 3 ou 4 atomes de carbone, par un radical alcoylthio ou benzylthio, par un radical phénoxy (éventuellement substitué par un atome de fluor ou substitué par un atome de chlore ou de brome en position −2, ou substitué par un radical alcoyloxy ou par 1 ou 2 radicaux alcoyle) ou par un radical pyridyloxy ou alcoylpipéridyloxy, étant entendu que, lorsque Het représente un radical quinolyle-2, R est autre que phényle et que les radicaux ou portions alcoyles cités ci-dessus sont droits ou ramifiés et contiennent 1 à 4 atomes de carbone,
caractérisé en ce que l'on fait agir un acide de formule générale:

R-COOH

dans laquelle R est défini comme ci-dessus ou un dérivé réactif de cet acide sur une amine de formule générale:

$H_2N$-Het

dans laquelle Het est défini comme précédemment, puis le cas échéant élimine les radicaux protecteurs, ou bien
pour préparer un produit pour lequel le symbole Het contient un substituant hydroxyméthyle, l'on réduit l'aldéhyde correspondant, ou bien
pour préparer un produit pour lequel le symbole R est un radical tétrahydropyridyle, l'on fait agir la tétrahydropyridine sur un carbamate de formule générale:

$C_6H_5OCONH$-Het

dans laquelle le symbole Het est défini comme ci-dessus.

**Claims for the Contracting States:**
BE, CH, DE, FR, GB, IT, LI, LU, NL, SE

1. A substituted amide derivative, characterized in that it corresponds to the general formula:

R-CONH-Het

in which the symbol R denotes a cycloalkyl radical containing 3 to 6 carbon atoms, a cyclohexadienyl or phenyl radical, or a phenyl radical substituted with 1 or 2 fluorine atoms or with a hydroxy radical, or substituted at the 3- or 4-position with an alkyl or alkyloxy radical, or at the 3- and 4-positions with a methylenedioxy radical, or substituted at the 2- or 3-position with a dialkylamino radical, or alternatively the symbol R denotes a heterocyclic radical

chosen from 3-pyridyl, alkyloxy-3-pyridyl, thienyl, alkylthienyl, furyl, tetrahydropyridyl, pyridazinyl and alkylpyridazinyl, and the symbol Het denotes a 2-quinolyl or 1,8-naphthyridin-2-yl radical optionally substituted at the 7-position with a halogen atom, with a hydroxymethyl radical, with an alkyl, alkyloxy or alkyloxyalkyloxy radical, with an alkenyloxy or alkynyloxy radical containing 3 or 4 carbon atoms, with an alkylthio or benzylthio radical, with a phenoxy radical (optionally substituted with a fluorine atom or substituted with a chlorine or bromine atom at the 2-position, or substituted with an alkyloxy radical or with 1 or 2 alkyl radicals), or with a pyridyloxy or alkylpiperidyloxy radical, with the proviso that, when Het denotes a 2-quinolyl radical, R is other than phenyl, and that the alkyl radicals and portions mentioned above are straight or branched and contain 1 to 4 carbon atoms.

2. An amide derivative according to claim 1, in which the symbol R is a cyclopropyl, cyclobutyl, 1,4-cyclohexadien-1-yl or phenyl radical, or a phenyl radical substituted with 1 or 2 fluorine atoms or with a hydroxy radical, or substituted at the 3- or 4-position with an alkyl radical containing 1 or 2 carbon atoms or an alkyloxy radical containing 1 to 4 carbon atoms, or at the 3- end 4-positions with a methylenedioxy radical or substituted with a dimethylamino radical at the 3-position, or alternatively the symbol R is a heterocyclic radical chosen from 3-pyridyl, 6-alkyloxy-3-pyridyl, thienyl, 5-alkylthienyl, furyl, 3-pyridazinyl or 6-alkyl-3-pyridazinyl in which the alkyl portions contain 1 or 2 carbon atoms, and the symbol Het denotes a 2-quinolyl or 1,8-naphthyridin-2-yl radical optionally substituted at the 7-position with a halogen atom, with a hydroxymethyl radical, with an alkyl or alkylthio radical containing 1 or 2 carbon atoms, with an alkyloxy radical containing 1 to 4 carbon atoms or an alkyloxyalkyloxy radical containing 2 to 4 carbon atoms, with an allyloxy, propargyloxy or benzylthio radical, with a phenoxy radical (optionally substituted with a fluorine atom or with a chlorine or bromine atom at the 2-position, with an alkyloxy radical or with 1 or 2 alkyl radicals containing 1 or 2 carbon atoms) or with a 3-pyridyloxy or 1-alkyl-4-piperidyloxy radical, with the proviso that, when the symbol Het denotes a 2-quinolyl radical, R is other than phenyl.

3. An amide derivative according to claim 1, in which the symbol R is a cyclopropyl, 1,4-cyclohexadien-1-yl or phenyl radical, or a phenyl radical substituted with 1 or 2 fluorine atoms or with a hydroxy radical, or substituted at the 3- or 4-position with an alkyloxy radical containing 1 or 2 carbon atoms or at the 3- and 4-positions with a methylenedioxy radical, or substituted at the 3-position with a dimethylamino radical, or alternatively the symbol R is a heterocyclic radical chosen from 3-pyridyl, 6-alkyloxy-3-pyridyl in which the alkyl portion contains 1 or 2 carbon atoms, thienyl or furyl, and the symbol Het denotes a 1,8-naphthyridin-2-yl radical optionally substituted at the 7-position with a halogen atom, with an alkyl, alkyloxy or alkylthio radical containing 1 or 2 carbon atoms, with an alkyloxyalkyloxy radical containing 2 to 4 carbon atoms, with an allyloxy or phenoxy radical (optionally substituted with a fluorine atom, or with a chlorine or bromine atom at the 2-position, with an alkyloxy radical or with 1 or 2 alkyl radicals containing 1 or 2 carbon atoms), or with a 3-pyridyloxy radical.

4. An amide derivative according to claim 1, in which the symbol R is a cyclopropyl or phenyl radical or a phenyl radical substituted at the 3- or 4-position with a fluorine atom, or substituted with 2 fluorine atoms or with a hydroxy radical, or substituted at the 3- or 4-position with a methoxy radical, or alternatively the symbol R is a heterocyclic radical chosen from 6-alkyloxy-3-pyridyl in which the alkyl portion contains 1 or 2 carbon atoms, 2-thienyl or 3-furyl, and the symbol Het denotes a 1,8-naphthyridin-2-yl radical substituted at the 7-position with a halogen atom, with a methoxy radical or with a phenoxy radical (optionally substituted with a fluorine atom or at the 2-position with a chlorine atom or a methyl radical, or at the 2- or 3-position with a methoxy radical), or the symbol R denotes a 1,4-cyclohexadien-1-yl radical, a phenyl radical (substituted at the 3- and 4-positions with a methylenedioxy radical or substituted at the 3-position with a dimethylamino radical) or a 3-pyridyl radical, and the symbol Het denotes a 1,8-naphthyridin-2-yl radical substituted at the 7-position with a halogen atom.

5. A process for preparing a product according to claim 1, wherein an acid of general formula:

R-COOH

in which R is defined as in claim 1, or a reactive derivative of this acid, is reacted with an amine of general formula:

$H_2N$-Het

in which Het is defined as in claim 1, and then, where appropriate, the protective radicals are removed.

6. A process according to claim 2, wherein a reactive derivative of the acid, chosen from the acid anhydride, a mixed anhydride, an acid halide or an ester, is reacted.

7. A process for preparing a product according to claim 1, for which the symbol Het contains a hydroxymethyl substituent, wherein the corresponding aldehyde is reduced.

8. A process for preparing a product according to claim 1, for which the symbol R is a tetrahydropyridyl radical, wherein tetrahydropyridine is reacted with a carbamate of general formula:

$C_6H_5OCONH$-Het

in which the symbol Het is defined as in Claim 1.

9. A pharmaceutical composition, which comprises at least one product according to claim 1, in the pure state or in combination with any other diluent or adjuvant which is compatible and pharmaceutically acceptable.

**Claims for the Contracting State:** AT

A process for preparing a new substituted amide derivative of general formula:

R-CONH-Het

in which the symbol R denotes a cycloalkyl radical containing 3 to 6 carbon atoms, a cyclohexadienyl or phenyl radical, or a phenyl radical substituted with 1 or 2 fluorine atoms or with a hydroxy radical, or substituted at the 3- or 4-position with an alkyl or alkyloxy radical, or at the 3- and 4-positions with a methylenedioxy radical, or substituted at the 2- or 3-position with a dialkylamino radical, or alternatively the symbol R denotes a heterocyclic radical chosen from 3-pyridyl, alkyloxy-3-pyridyl, thienyl, alkylthienyl, furyl, tetrahydropyridyl, pyridazinyl and alkylpyridazinyl, and the symbol Het denotes a 2-quinolyl or 1,8-naphthyridin-2-yl radical optionally substituted at the 7-position with a halogen atom, with a hydroxymethyl radical, with an alkyl, alkyloxy or alkyloxyalkyloxy radical, with an alkenyloxy or alkynyloxy radical containing 3 or 4 carbon atoms, with an alkylthio or benzylthio radical, with a phenoxy radical (optionally substituted with a fluorine atom or substituted with a chlorine or bromine atom at the 2-position, or substituted with an alkyloxy radical or with 1 or 2 alkyl radicals), or with a pyridyloxy or alkylpiperidyloxy radical, with the proviso that, when Het denotes a 2-quinolyl radical, R is other than phenyl, and that the alkyl radicals and portions mentioned above are straight or branched and contain 1 to 4 carbon atoms,
wherein an acid of general formula:

R-COOH

in which R is defined as above, or a reactive derivative of this acid , is reacted with an amine of general formula:

$H_2N$-Het

in which Het is defined as above, and then, where appropriate, the protective radicals are removed, or alternatively to prepare a product for which the symbol Het contains a hydroxymethyl substituent, the corresponding aldehyde is reduced, or alternatively to prepare a product for which the symbol R is a tetrahydropyridyl radical, tetrahydropyridine is reacted with a carbamate of general formula:

$C_6H_5OCONH$-Het

in which the symbol Het is defined as above.

**Patentansprüche für die Vertragsstaaten:**
BE, CH,DE,FR, GB, IT, LI, LU, NL, SE

1. Ein substituiertes Amidderivat, dadurch gekennzeichnet, dass es der allgemeinen Formel

R-CONH-Het

entspricht, worin das Symbol R bedeutet einen Cycloalkylrest mit 3 bis 6 Kohlenstoffatomen, Cyclohexadienyl, Phenyl, Phenyl substituiert durch 1 oder 2 Fluoratome oder einen Hydroxyrest, oder substituiert in 3- oder 4-Stellung durch einen Alkyl- oder Alkoxyrest, oder in 3- und 4-Stellung durch einen Methylendioxyrest, oder substituiert in 2- oder 3-Stellung durch einen Dialkylaminorest, oder das Symbol R bedeutet einen Heterocyclylrest, ausgewählt unter Pyridyl-3, Alkyloxypyridyl-3, Thienyl, Alkylthienyl, Furyl, Tetrahydropyridyl, Pyridazinyl und Alkylpyridazinyl, und das Symbol Het bedeutet einen Chinolyl-2-Rest oder 1,8-Naphthyridin-yl-2-Rest, gegebenenfalls substituiert in 7-Stellung durch ein Halogenatom, durch einen Hydroxymethylrest, durch einen Alkyl-, Alkyloxy-, Alkyloxyalkyloxyrest, durch einen Alkenyloxy- oder Alkinyloxyrest mit 3 oder 4 Kohlenstoffatomen, durch einen Alkylthio- oder Benzylthiorest, durch einen Phenoxyrest (gegebenenfalls substituiert durch ein Fluoratom oder substituiert durch ein Chlor- oder Bromatom in 2-Stellung, oder substituiert durch einen Alkyloxyrest oder durch 1 oder 2 Alkylreste), oder durch einen Pyridyloxy- oder Alkylpiperidyloxyrest, wobei jedoch, falls Het einen Chinolyl-2-Rest bedeutet, R anders als Phenyl ist, und dass die vorstehend erwähnten Alkylreste und Alkylteile gerade oder verzweigt sind und 1 bis 4 Kohlenstoffatome enthalten.

2. Amidderivat gemäss Anspruch 1, dadurch gekennzeichnet, dass das Symbol R ein Cyclopropyl-, Cyclobutyl-, 1,4-Cyclohexadien-1-, Phenylrest, Phenyl substituiert durch 1 oder 2 Fluoratome oder durch einen Hydroxyrest, oder substituiert in 3- oder 4-Stellung durch einen Alkylrest mit 1 oder 2 Kohlenstoffatomen oder Alkoxyrest mit 1 bis 4 Kohlenstoffatomen, oder in 3- und 4-Stellung durch einen Methylendioxyrest oder substituiert durch einen Dimethylaminorest in 3-Stellung, ist, oder das Symbol R ist ein Heterocyclylrest, ausgewählt unter Pyridyl-3, 6-Alkyloxy-pyridyl-3, Thienyl, 5-Alkylthienyl, Furyl, Pyridazinyl-3 oder 6-Alkylpyridazinyl-3, deren Alkylteile 1 oder 2 Kohlenstoffatome enthalten und das Symbol Het bedeutet einen Chinolyl-2- oder 1,8-Naphthyridin-yl-2-Rest, gegebenenfalls substituiert in 7-Stellung durch ein Halogenatom, durch einen Hydroxymethylrest, durch einen Alkyl- oder Alkylthiorest mit 1 oder 2 Kohlenstoffatomen, durch einen Alkyloxyrest mit 1 bis 4 Kohlenstoffatomen, oder Alkyloxyalkyloxyrest mit 2 bis 4 Kohlenstoffatomen, durch einen Allyloxy-, Propargyloxy- oder Benzylthiorest, durch einen Phenoxyrest (gegebenenfalls substituiert durch ein Fluoratom oder durch ein Chlor- oder Bromatom in 2-Stellung, durch einen Alkyloxyrest oder durch 1 oder 2 Alkylreste mit 1 oder 2 Kohlenstoffatomen) oder durch einen Pyridyl-3-oxy- oder 1-Alkyl-piperidyl-4-oxyrest, wobei, falls das Symbol Het einen Chinolyl-2-Rest bedeutet, R anders als Phenyl ist.

3. Amidderivat gemäss Anspruch 1, dadurch gekennzeichnet, dass das Symbol R ist ein Cyclopropyl-, 1,4-Cyclohexadien-yl-1-, Phenylrest, Phenylrest substituiert durch 1 oder 2 Fluoratome oder durch einen Hydroxyrest, oder substituiert in 3- oder 4-Stellung durch einen Alkyloxyrest mit 1 oder 2 Kohlenstoffatomen oder in 3- und 4-Stellung durch

einen Methylendioxyrest, oder substituiert in 3-Stellung durch einen Dimethylaminorest, oder aber das Symbol R ist ein Heterocyclylrest, ausgewählt unter Pyridyl-3-, 6-Alkyloxy-pyridyl-3, dessen Alkylteil 1 oder 2 Kohlenstoffatome hat, Thienyl oder Furyl und das Symbol Het bedeutet einen 1,8--Naphthyridin-yl-2-Rest, gegebenenfalls substituiert in 7-Stellung durch ein Halogenatom, durch einen Alkyl-, Alkyloxy- oder Alkylthiorest mit 1 oder 2 Kohlenstoffatomen, durch einen Alkyloxyalkyloxyrest mit 2 bis 4 Kohlenstoffatomen, durch einen Allyloxy- oder Phenoxyrest (gegebenenfalls substituiert durch ein Fluoratom oder durch ein Chlor- oder Bromatom in 2-Stellung, durch einen Alkoxyrest oder durch 1 oder 2 Alkylreste mit 1 oder 2 Kohlenstoffatomen), oder durch einen Pyridyl-3-oxyrest.

4. Amidderivat gemäss Anspruch 1, dadurch gekennzeichnet, dass das Symbol R ist ein Cyclopropyl-, Phenylrest, Phenylrest substituiert in 3- oder 4-Stellung durch ein Fluoratom, oder substituiert durch 2 Fluoratome oder durch einen Hydroxyrest, oder substituiert in 3- oder 4-Stellung durch einen Methoxyrest, oder aber das Symbol R ist ein Heterocyclylrest, ausgewählt unter 6-Alkyloxy-pyridyl-3, dessen Alkylteil 1 oder 2 Kohlenstoffatome enthält, Thienyl-2 oder Furyl-3, und das Symbol Het bedeutet einen 1,8-Naphthyridin-yl-2-Rest, substituiert in 7-Stellung durch ein Halogenatom, durch einen Methoxyrest oder durch einen Phenoxyrest (gegebenenfalls substituiert durch ein Fluoratom oder in 2-Stellung durch ein Chloratom oder einen Methylrest oder in 2- oder 3-Stellung durch einen Methoxyrest), oder das Symbol R bedeutet einen 1,4-Cyclohexadienyl-yl-1-Rest, Phenyl (substituiert in 3- und 4-Stellung durch einen Methylendioxyrest oder substiuiert in 3-Stellung durch einen Dimethylaminorest) oder einen Pyridyl-3-Rest und das Symbol Het bedeutet einen 1,8-Naphthyridin-yl-2-Rest, substituiert in 7-Stellung durch ein Halogenatom.

5. Verfahren zur Herstellung eines Produkts gemäss Anspruch 1, dadurch gekennzeichnet, dass man eine Säure der allgemeinen Formel

R-COOH

worin R wie in Anspruch 1 definiert ist, oder ein reaktionsfähiges Derivat dieser Säure auf ein Amin der allgemeinen Formel

$H_2N$-Het

worin Het wie in Anspruch 1 definiert ist, einwirken lässt und gegebenenfalls die Schutzreste entfernt.

6. Verfahren gemäss Anspruch 2, dadurch gekennzeichnet, dass man ein reaktionsfähiges Derivat der Säure, ausgewählt unter dem Säureanhydrid, einem gemischten Anhydrid, einem Säurehalogenid oder einem Ester, einwirken lässt.

7. Verfahren zur Herstellung eines Produktes gemäss Anspruch 1, wobei das Symbol Het einen Hydroxymethylsubstituenten enthält, dadurch gekennzeichnet, dass man den entsprechenden Aldehyd reduziert.

8. Verfahren zur Herstellung eines Produkts gemäss Anspruch 1, wobei das Symbol R ein Tetrahydropyridylrest ist, dadurch gekennzeichnet, dass man das Tetrahydropyridin auf ein Carbamat der allgemeinen Formel

$C_6H_5OCONH$-Het

worin das Symbol Het wie in Anspruch 1 definiert ist, einwirken lässt.

9. Pharmazeutische Zusammensetzung, dadurch gekennzeichnet, dass sie mindestens ein Produkt gemäss Anspruch 1 in reinem Zustand oder in Assoziation mit jedem anderen verträglichen und pharmazeutisch annehmbaren Verdünnungs- oder Hilfsmittel enthält.

**Patentanspruch für den Vertragsstaat:** AT

Verfahren zur Herstellung eines neuen substituierten Amidderivats der allgemeinen Formel:

R-CONH-Het

in welcher das Symbol R ein Cycloalkylrest mit 3 bis 6 Kohlenstoffatomen, ein Cyclohexadienylrest, ein Phenylrest, ein durch 1 oder 2 Fluoratome oder einen Hydroxyrest, substituierter oder in 3- oder 4-Stellung durch einen Alkyl- oder Alkoxyrest substituierter oder in 3- und 4-Stellung durch einen Methylendioxyrest oder in 2- oder 3-Stellung durch einen Dialkylaminorest, substituierter Phenylrest ist, oder das Symbol R bedeutet einen heterocyclischen Rest ausgewählt aus 3-Pyridyl, 3-Alkyloxypyridyl, Thienyl, Alkylthienyl, Furyl, Tetrahydropyridyl, Pyridazinyl und Alkylpyridazinyl, und das Symbol Het ein gegebenenfalls in 7-Stellung durch ein Halogenatom, durch einen Hydroxymethylrest, durch einen Alkyl-, Alkyloxy-, Alkyloxyalkyloxyrest, durch einen Alkenyloxy- oder Alkinyloxyrest mit 3 oder 4 Kohlenstoffatomen, durch einen Alkylthio- oder Benzylthiorest, durch einen Phenoxyrest (gegebenenfalls substituiert durch ein Fluoratom oder substituiert durch ein Chlor- oder Bromatom in 2-Stellung oder durch einen Alkyloxyrest oder 1 oder 2 Alkylreste) oder durch einen Pyridyloxy- oder Alkylpiperidyloxyrest substituierten 2-Chinolyl- oder 1,8-Naphthyridin-2-ylrest bedeutet, wobei R eine andere Bedeutung als Phenyl hat, wenn Het ein 2-Chinolylrest ist, und die oben angegebenen Alkylreste und -teile gerade oder verzweigt sind und 1 bis 4 Kohlenstoffatome enthalten, dadurch gekennzeichnet, dass man eine Säure der allgemeinen Formel:

R-COOH

in welcher R wie oben definiert ist, oder reaktives Derivat dieser Säure mit einem Amin der allgemeinen Formel:

$H_2N$-Het

in welcher Het wie vorstehend definiert ist, umsetzt,

worauf man zutreffendenfalls die Schutzgruppe entfernt, oder dass man zur Herstellung eines Produktes, bei welchem das Symbol Het einen Hydroxymethylsubstituenten enthält, den entsprechenden Aldehyd reduziert oder dass man zur Herstellung eines Produktes, bei welchem das Symbol R ein Tetrahydropyridylrest ist, das Tetrahydropyridin mit einem Carbonat der allgemeinen Formel:

$$C_6H_5OCONH\text{-}Het$$

in welcher das Symbol Het wie oben definiert ist, umsetzt.